(19) Europäisches Patentamt
European Patent Office
Office européen des brevets

(11) EP 3 781 218 B1

(12) # EUROPÄISCHE PATENTSCHRIFT

(45) Veröffentlichungstag und Bekanntmachung des Hinweises auf die Patenterteilung:
**18.09.2024 Patentblatt 2024/38**

(21) Anmeldenummer: **19728898.8**

(22) Anmeldetag: **17.05.2019**

(51) Internationale Patentklassifikation (IPC):
***A61L 2/18*** *(2006.01)* ***A61L 2/00*** *(2006.01)*
***A61L 2/14*** *(2006.01)*

(52) Gemeinsame Patentklassifikation (CPC):
**A61L 2/186; A61L 2/0088; A61L 2/14;**
A61L 2202/11; A61L 2202/14

(86) Internationale Anmeldenummer:
**PCT/EP2019/062897**

(87) Internationale Veröffentlichungsnummer:
**WO 2019/219959 (21.11.2019 Gazette 2019/47)**

(54) **DESINFEKTIONSVERFAHREN MIT EINEM DURCH REAKTION VON H2O2 UND NO2- IN SITU GEBILDETEM DESINFEKTIONSWIRKSTOFF**

DISINFECTION PROCESS USING AN ACTIVE DISINFECTING SUBSTANCE FORMED IN SITU BY REACTING H2O2 AND NO2-

PROCÉDÉ DE DÉSINFECTION UTILISANT UNE SUBSTANCE ACTIVE DÉSINFECTANTE FORMÉE IN SITU PAR RÉACTION ENTRE H2O2 ET NO2-

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.05.2018 PCT/EP2018/063226**

(43) Veröffentlichungstag der Anmeldung:
**24.02.2021 Patentblatt 2021/08**

(73) Patentinhaber: **Nebula Biocides GmbH**
**17489 Greifswald (DE)**

(72) Erfinder:
- **SCHMIDT-BLEKER, Ansgar**
  **33729 Bielefeld (DE)**
- **WINTER, Jörn**
  **17489 Greifswald (DE)**
- **WELTMANN, Klaus-Dieter**
  **18609 Ostseebad Binz (DE)**
- **BENDT, Hannes**
  **17493 Greifswald (DE)**

(74) Vertreter: **Schulz Junghans**
**Patentanwälte PartGmbB**
**Großbeerenstraße 71**
**10963 Berlin (DE)**

(56) Entgegenhaltungen:
**EP-A1- 3 346 808 US-A1- 2017 172 149**
**US-B2- 8 871 146**

- **W. HEASELGRAVE ET AL: "Acidified nitrite enhances hydrogen peroxide disinfection of Acanthamoeba, bacteria and fungi", JOURNAL OF ANTIMICROBIAL CHEMOTHERAPY., vol. 65, no. 6, 23 March 2010 (2010-03-23), GB, pages 1207 - 1214, XP055547681, ISSN: 0305-7453, DOI: 10.1093/jac/dkq075**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 781 218 B1

**Beschreibung**

Stand der Technik

Die vorliegende Offenbarung betrifft ein Verfahren (beansprucht) und eine Vorrichtung (nicht Teil der Ansprüche) zur Desinfektion von Oberflächen, insbesondere zur Desinfektion von Körperteilen, insbesondere von Händen.

Die biozide Wirkung der Reaktionsprodukte von Wasserstoffperoxid ($H_2O_2$) und Nitrit ($NO_2^-$) unter Zugabe einer Säure ist bereits in der Literatur bekannt. Ebenfalls in der Literatur bekannt ist, dass bei dieser Reaktion eine Vielzahl reaktiver Spezies, unter anderem Peroxinitritsäure (ONOOH) und ihr Säurerest-Ion Peroxinitrit ($ONOO^-$), Stickstoffmonoxid (NO), Stickstoffdioxid ($NO_2$), Hydroxyl-Radikale (OH) und Hydroperoxyl-Radikale ($HO_2$) gebildet werden (Fig. 1). Insbesondere Peroxinitritsäure wird in der Literatur als stark biozid angenommen, hat jedoch eine sehr kurze Lebensdauer von nur 0,8 s, was ihre Anwendung als desinfizierendes Mittel problematisch gestaltet, da eine Mindestwirkdauer eingehalten werden muss, um eine ausreichend große Anzahl an Keimen abzutöten.

$$\mathrm{H_2O_2} + \mathrm{NO_2^-} \rightarrow \mathrm{Reaktionsprodukte} \qquad (1)$$

Die Reaktionsrate der Reaktion (1) ist abhängig vom pH-Wert der Lösung und wurde in verschiedenen Arbeiten untersucht (Anbar & Taube, 1954; Damschen & Martin, 1983; E. Halfpenny, 1952; Lee & Lind, 1986; Lukes et al., 2015; Vione et al., 2003). Die Zwischenschritte der Reaktion (1) sind nicht im Detail bekannt. In (Vione et al., 2003) werden als Zwischenschritte für Reaktion (1) die Reaktionen

$$\mathrm{H_2O_2} + \mathrm{H_3O^+} \leftrightarrow \mathrm{H_3O_2^+} + \mathrm{H_2O} \qquad (2)$$

$$\mathrm{NO_2^-} + \mathrm{H_3O^+} \leftrightarrow \mathrm{HNO_2} + \mathrm{H_2O} \qquad (3)$$

$$\mathrm{H_3O_2^+} + \mathrm{HNO_2} \rightarrow \mathrm{ONOOH} + \mathrm{H_3O^+} \qquad (4)$$

als möglicher Bildungsweg für ONOOH vorgeschlagen.

In Fig. 1 ist eine Auswahl chemischer Prozesse dargestellt, welche gemäß eines im Rahmen der Erfindung erstellten chemischen Modells in Folge der Vermischung von $H_2O_2$ und $NO_2^-$ zur Bildung von reaktiven Spezies führen.

Gleichzeitig entstehen durch die Reaktion von $H_2O_2$ und $NO_2^-$ jedoch auch in Wasser gelöste Stickoxide ($NO_x$). Bei $NO_x$ handelt es sich um übelriechende und gesundheitsgefährdende Gase. Es ist auch bekannt, dass $NO_x$ nicht in hohem Maße in Flüssigkeiten lösbar sind und infolgedessen aus Flüssigkeiten ausgasen können. Diese Ausgasung ist insbesondere in Flüssigkeitsfilmen mit einer großen Oberfläche relevant. Im Gegensatz zu den kleineren Flüssigkeitsoberflächen in Suspensionsversuchen ist die Ausgasung bei der Desinfektion von realen Oberflächen ein zu berücksichtigender Faktor und erschwert daher die Nutzung der Reaktion (1) für die Desinfektion.

In der Literatur ist beschrieben, dass Mischungen aus $NO_2^-$ und $H_2O_2$ antimikrobiell wirksam sind, und bei einer Einwirkdauer von mehreren Stunden (Jiang & Yuan, 2013) oder mehr als 15 Minuten (Heaselgrave, Andrew, & Kilvington, 2010) zu einer signifikanten Reduktion von Mikroorganismen in Suspension führen können. Hierbei wird angenommen, dass ONOOH ein für die Dekontaminationswirkung wichtiger Bestandteil ist. Es entstehen durch die Reaktion von $H_2O_2$ und $NO_2^-$ noch weitere antimikrobiell wirksame Spezies, wie beispielsweise NO, $NO_2$, OH, und $HO_2$. Auch ist bekannt, dass die Vermischung von $NO_2^-$ und einer Säure auch zu einer sporiziden Wirkung führt (Szabo, Adcock, & Rice, 2014). Aus diesen Arbeiten ist bekannt, dass sich eine Ansäuerung der Suspension für die Dekontamination mittels $NO_2^-$ oder einer Mischung aus $H_2O_2$ und $NO_2^-$ vorteilhaft auf die Desinfektionswirkung auswirkt.

Die Arbeit von Heaselgrave, Andrew, & Kilvington, 2010 schlägt eine Kombination aus $H_2O_2$ und $NO_2^-$ vor, um Kontaktlinsen zu desinfizieren. Hierin verwenden die Autoren eine Desinfektionslösung, welche 171 mmol $H_2O_2$ und 29 mmol $NO_2^-$ bei einem pH-Wert von 5 enthält. Der kürzeste Einwirkzeitraum, welchen die Autoren angeben, um eine desinfizierende Wirkung zu erhalten, ist hierbei 18 Minuten. Für eine Oberflächendesinfektion und insbesondere für eine hygienische Handdesinfektion wäre dieser Einwirkzeitraum nicht zufriedenstellend. Ein wirksameres Verfahren kann trivialer Weise durch Erhöhung der Konzentrationen von $NO_2^-$ und $H_2O_2$ erreicht werden. Die vorliegende Erfindung richtet sich jedoch auf ein Verfahren, welches bei einem limitierten Einsatz von $NO_2^-$ eine Abtötung von unbehüllten Viren und Bakteriensporen in ebenfalls stark limitierter Zeit ermöglicht und bei welchem keine langanhaltende Toxizität

gegeben ist.

Hierbei ist ein Verfahren hinsichtlich des Ressourceneinsatzes optimal, welches bei gegeben Rahmenbedingungen möglichst eine möglichst hohe Effizienz aufweist, wobei die Effizienz als Quotient der jeweiligen Wirksamkeit und der theoretisch maximal erreichbaren Wirksamkeit zu verstehen ist.

Lukes et al 2015 beschreibt ebenfalls die Verwendung von Wasserstoffperoxid und Nitrit zur Desinfektion. Dabei wird eine Versuchsanordnung bei einem pH-Wert von 3,3 mit 200 $\mu$M $H_2O_2$ und 90 $\mu$M $NO_2^-$ beschrieben. Auch hier beträgt die notwendige Einwirkzeit mehrere Stunden.

Analoges gilt für die WO 2011/134010 A1, welche die Verwendung von angesäuertem $NO_2^-$ in Abwässer über einen Zeitraum von einer Stunde bis mehreren Tagen beschreibt.

In Ikawa et al., 2016 ist ein Desinfektionsverfahren beschrieben, bei dem Wasser mit Plasma behandelt wird, und anschließend mit einer Bakteriensuspension versehen wird. Die desinfizierende Wirkung ist hier also auf die durch das Plasma entstehenden reaktiven Spezies zurückzuführen. Gemäß der Studie bildet sich hier vor allem Peroxosalpetersäure. Ein weiteres Desinfektionsverfahren von Ikawa et al. In US 8,871,146 beschreibt ein Verfahren zur Sterilisation von Mikroorganismen in oder auf einer Oberfläche oder Flüssigkeit. Hierbei wird Plasma in einem atmosphärischen Stickstoffgas generiert, welches wiederum $O_2^-$ Radikale generiert welche mit den Protonen einer sauren Lösung zu Hydroperoxidradikalen reagieren. In der Luft vorkommendes Stickstoff und Sauerstoff formen NO welches zusammen mit den Hydroperoxidradikalen die hochgradig anti-mikrobielle Verbindung, Peroxynitrit bilden. Das Desinfektionsverfahren von Kitano et al. ist in US 20170172149 auch für Oberflächen beschrieben. Es basiert auch auf der desinfizierenden Wirkung einer Mischung aus Peroxid und Nitrit. Allerdings wird hier als desinfizierende Spezies Peroxosalpetersäure gebildet, welche bei pH-Werten kleiner 2 (pH=0) entsteht. Insbesondere der saure Bereich (pH = 0) ist für die Bildung der desinfizierenden Spezies sehr wichtig, sodass es teilweise notwendig ist, den pH-Wert nachträglich wieder anzuheben (auf pH=4,8), um auch pH-Wert empfindliche Oberflächen desinfizieren zu können. Die Halbwertszeit der desinfizierenden Spezies, Peroxosalpetersäure, wird dort mit etwa 10 Minuten bei Raumtemperatur, in anderen Dokumenten (Lammel et al., 1990) bei Raumtemperatur mit ca. 3 min angegeben. Die desinfizierenden Spezies in der vorliegenden Erfindung hingegen zerfallen bei Raumtemperatur in einer sehr viel kürzeren Zeit (Halbwertszeiten von ca. 1s), so dass ausgeschlossen ist, dass es sich hierbei um die gleichen aktiven Substanzen handelt.

Alle oben genannten Untersuchungen beziehen sich auf die Reduktion von Mikroorganismen in Suspension unter Anwendung längerer Expositionszeiten, insbesondere länger als 15 Minuten. Es ist nach unserem Kenntnisstand jedoch kein Verfahren bekannt, bei dem eine desinfizierende Wirkung durch Mischung von $H_2O_2$ und $NO_2^-$ bereits innerhalb weniger Minuten oder Sekunden erfolgt und bei dem eine Desinfektion nicht in Suspension, sondern auf Oberflächen stattfindet.

Daher zielt das im Folgenden dargestellte Verfahren auf die Dekontamination von Oberflächen mittels Mischungen aus $NO_2^-$ und $H_2O_2$ in sehr kurzer Zeit ab, wobei eine Wirklösung durch Mischung von $N0_2^-$ und $H_2O_2$ gebildet wird, und in einem Verteilungsschritt auf eine Oberfläche aufgebracht und/oder dort verteilt wird oder sich durch Diffusion dort verteilt.

## Aufgabe der Erfindung

Es ergibt sich also das Problem, ein Verfahren zur Oberflächendesinfektion bereit zu stellen, welches auf den Ausgangsstoffen $H_2O_2$ und $NO_2^-$ basiert, die über einen Verteilungsschritt auf der zu desinfizierenden Oberfläche aufgebracht und/oder verteilt werden, und dessen gesamte Prozessdauer deutlich kleiner ist, als bei bisher bekannten Verfahren auf Basis der Mischung von $H_2O_2$ und $NO_2^-$ Gleichzeitig muss das Verfahren Oberflächen mit puffernder Wirkung tolerieren, die $NO_x$-Emissionen möglichst weit unter den als gesundheitsschädlich angenommenen Konzentrationen halten und auch dann noch zuverlässig funktionieren, wenn ein Teil des eingesetzten $NO_2^-$ als $NO_x$ ausgast.

Eine Aufgabe der Erfindung ist weiter, ein Desinfektionsverfahren bereit zu stellen, bei dem durch Reaktion von $H_2O_2$ und $NO_2^-$ eine Wirklösung entsteht, die zur Desinfektion von Oberflächen verwendet werden kann.

Eine weitere Aufgabe der vorliegenden Offenbarung ist die Bereitstellung einer Vorrichtung, mit der die nötigen Reagenzien für die Anwendung in einem Desinfektionsverfahren mit einer in situ hergestellten Wirklösung vorgehalten, zusammengeführt, und auf die zu desinfizierende Oberfläche gebracht werden können.

Die Aufgabe der Erfindung wird durch den Gegenstand des unabhängigen Verfahrensanspruchs 1 gelöst Vorteilhafte Ausführungsformen des Verfahrens sind in den Unteransprüchen angegeben. Diese und weitere Ausführungsformen werden im Folgenden beschrieben.

## Definitionen

Gemäß der vorliegenden Erfindung umfasst die *Wirklösung* eine Desinfektionslösung, die auf der zu desinfizierenden Oberfläche angewendet wird. Hierbei bezeichnet die Oberfläche eine plane Fläche oder eine Fläche mit Unebenheiten und/oder Hohlräumen. Die Wirklösung kann, zusätzlich zu den in situ gebildeten desinfizierenden Wirkstoffen, noch Zusatzstoffe enthalten. Solche Zusatzstoffe umfassen unter anderem, aber nicht ausschließlich, Lösungsmittel, Pufferlösungen, Basen, Duftstoffe, Farbstoffe und/oder weitere Desinfektionsmittel und/oder Ozon, sowie weitere Reaktionsprodukte und reaktive Zwischenstufen der Reaktion zwischen $H_2O_2$ und $NO_2^-$.

Gemäß der vorliegenden Erfindung umfasst ein *Verdünnungsschritt* die Verdünnung der Edukte mit Lösungsmitteln und/oder Zusatzstoffen. Der Verdünnungsschritt ist dem Vermischungsschritt vorangestellt oder erfolgt zeitgleich mit dem Vermischungsschritt. Gemäß der vorliegenden Erfindung umfasst ein *Vermischungsschritt* die Vermischung von Edukten zum Erhalt der Wirklösung. Während des Vermischungsschrittes können zusätzlich auch Zusatzstoffe zu den Edukten gemischt werden. Der Vermischungsschritt kann aus mehreren Teilschritten zusammengesetzt sein. Der Vermischungsschritt beginnt zum Zeitpunkt $t_0=0$.

Gemäß der vorliegenden Erfindung umfasst ein *Verteilungsschritt* die Verteilung der Wirklösung auf der zu desinfizierenden Oberfläche. Hierbei wird jeder Punkt auf der zu desinfizierenden Oberfläche mit Wirklösung benetzt. Der Verteilungsschritt kann gleichzeitig mit dem Vermischungsschritt zum Zeitpunkt to beginnen oder dem nachgestellt sein.

Gemäß der vorliegenden Erfindung umfasst der *Verarbeitungszeitraum* $Z_A$ den Vermischungs- und den Verteilungsschritt, also den Zeitraum, der benötigt wird, um die Edukte zum Erhalt der Wirklösung zu vermischen und jeden zu desinfizierenden Punkt auf der zu desinfizierenden Oberfläche mit Wirklösung zu benetzen. Der Verarbeitungszeitraum beginnt zum Zeitpunkt *to=0,* an dem die Edukte zuerst miteinander in Kontakt gebracht werden und endet zum Zeitpunkt $t_1$, an dem jeder Punkt auf der zu desinfizierenden Oberfläche mit Wirklösung benetzt ist. Der pH-Wert und die Temperatur können sich innerhalb des Verarbeitungszeitraums verändern insbesondere wenn die Vermischung vor dem ersten Kontakt mit der Oberfläche erfolgt und die Oberfläche den pH-Wert und/oder die Temperatur beeinflusst. Aus diesem Grund sind pH-Wert und Temperatur in während des Prozesses zeitabhängig.

Im Rahmen der Erfindung können auch vor dem Verarbeitungszeitraum, also vor dem Zeitpunkt *to=0,* für das Desinfektionsverfahren relevante Schritte, wie z.B. ein Verdünnungsschritt, ablaufen.

Gemäß der vorliegenden Erfindung umfasst ein *Einwirkschritt* die Einwirkung der Wirklösung auf der mit Wirklösung benetzten Oberfläche zur Desinfektion. Der Einwirkschritt wird durch den Einwirkzeitraum $Z_E$ beschrieben.

Gemäß der vorliegenden Erfindung umfasst der *Einwirkzeitraum* $Z_E$ den Zeitraum, der benötigt wird, um durch die Wirklösung eine ausreichende Desinfektionswirkung zu erzielen. Der Einwirkzeitraum beginnt zum Zeitpunkt $t_1$, an dem jeder Punkt der zu desinfizierenden Oberfläche mit Wirklösung benetzt ist, und endet zum Zeitpunkt $t_2$, an dem jeder Punkt der mit Wirklösung benetzten Oberfläche desinfiziert ist.

Gemäß der vorliegenden Erfindung ist $NO_2^-$ ein Nitritsalz mit der allgemeinen Formel $M_xNO_2$, wobei M ein Alkali- oder Erdalkalimetall ist und $x$=1 oder $x$=2 sein kann. Insbesondere ist M Natrium oder Kalium und $x$=1. Das Nitritsalz kann dabei als Salz in Lösung oder als Feststoff vorliegen. Hierbei liegt $NO_2^-$ in Lösung je nach pH-Wert als Anion $NO_2^-$ oder als Säure $HNO_2$ vor.

Gemäß der vorliegenden Erfindung werden die Wirkstoffe, die die Reaktionsprodukte der Reaktion von $H_2O_2$ und $NO_2^-$, sind, in situ gebildet. In situ heißt, dass die Wirkstoffe erst bei Bedarf generiert werden.

Detaillierte Beschreibung der Erfindung

Das Desinfektionsverfahren der vorliegenden Erfindung aufweisend wenigstens die Edukte $H_2O_2$ und $NO_2^-$ besteht aus mehreren Teilschritten umfassend wenigstens:

- einen Vermischungsschritt, wobei die Edukte zum Erhalt einer Wirklösung vermischt werden;
- einen Verteilungsschritt, bei dem die Wirklösung auf einer zu desinfizierenden Oberfläche verteilt wird,

wobei der Vermischungsschritt und der Verteilungsschritt in einem Verarbeitungszeitraum $Z_A$ stattfinden, der zum Zeitpunkt *to* beginnt, wenn die Edukte zuerst miteinander in Kontakt gebracht werden, und zum Zeitpunkt $t_1$, bei dem jeder Punkt auf der zu desinfizierenden Oberfläche mit Wirklösung benetzt ist, endet, wobei *to* gleich 0 ist und $t_1$ größer als $t_0$ ist
und

- darauffolgend einen Einwirkschritt, bei dem die verteilte Wirklösung über einen Einwirkzeitraum $Z_E$, der zum Zeitpunkt $t_1$ beginnt und nach der Zeitspanne $Z_E$ zum Zeitpunkt $t_2$ endet, auf der mit Wirklösung kontaktierten Oberfläche einwirkt,

wobei $t_2$ den Zeitpunkt darstellt, an dem jeder Punkt auf der mit Wirklösung kontaktierten Oberfläche ausreichend lange mit Wirklösung benetzt ist, um eine desinfizierende Wirkung zu erhalten, und wobei $t_2$ größer als $t_1$ ist,

gekennzeichnet dadurch, dass

die maximale $NO_2^-$ Konzentration zum Zeitpunkt $t_0$ *des Vermischungsschritts* bei 300 mM liegt, und

die zeitintegrierte Reaktionsrate W über den Einwirkzeitraum $Z_E$ durch das Integral

$$W = \int_{t_1}^{t_2} k_1 \cdot [H_2O_2] \cdot [NO_2^-]\, dt \geq 10\, mM \quad (5)$$

dargestellt wird,

wobei $t_1$ und $t_2$ wie oben definiert sind, und

wobei $t_2$ 3 Minuten nicht übersteigt,

wobei $[H_2O_2]$ und $[NO_2^-]$ die Konzentrationen der Edukte während des Einwirkzeitraums $Z_E$ bezeichnen, und

wobei $k_1$ die abhängige Geschwindigkeitskonstante der Reaktion zwischen $H_2O_2$ und $NO_2^-$ bzw. $HNO_2$ bezeichnet,

wobei $k_1$ vom pH-Wert der Wirklösung auf der Oberfläche abhängt und

wobei der pH-Wert der Wirklösung auf der Oberfläche und die Temperatur eine Zeitabhängigkeit aufweisen können, und
wobei der pH-Wert der Wirklösung vor Kontakt mit der zu desinfizierenden Oberfläche und der pH-Wert der Wirklösung auf der mit Wirklösung kontaktierten Oberfläche im Bereich von $2{,}1 \leq pH < 6{,}8$ liegen.

Die pH-Wert-abhängige Geschwindigkeitskonstante $k_1$ kann folgendermaßen berechnet werden:

$$k_1 = k_4 \frac{[H_3O^+]^2}{\left(K_{S,H_3O_2^+} + [H_3O^+]\right)\left(K_{S,HNO_2} + [H_3O^+]\right)} \quad (6)$$

mit

$$k_4 = 3{,}56 \cdot 10^{14} \exp\left(-\frac{E_A}{RT}\right) M^{-1}s^{-1} \quad (7)$$

$$K_{S,HNO_2} = 5{,}13 \times 10^{-4} \quad (8)$$

$$K_{S,H_3O_2^+} = 2 \times 10^{-2} \quad (9)$$

und der einheitenlosen Größe

$$[H_3O^+] = 10^{-pH} \quad (10)$$

mit der effektiven Aktivierungsenergie $E_A$=70 kJ/mol und der Temperatur T. Bei 20 °C beträgt $k_4$ 120 $M^{-1}s^{-1}$.

Die zeitabhängigen Konzentrationen der Edukte $NO_2^-$ und $H_2O_2$ können während des Einwirkzeitraums mittels folgender Gleichungen berechnet werden:

$$[NO_2^-] = \frac{A}{k_1}, \quad (11)$$

$$[H_2O_2] = \frac{A+D}{k_1 + rk_1}, \quad (12)$$

mit

$$A = -\frac{D}{1-\exp(D(t-C))} \quad (13)$$

$$C = -\frac{\ln\left(\frac{D}{[NO_2^-]_0 \cdot k_1}+1\right)}{D} \quad (14)$$

und

$$D = [H_2O_2]_0 \cdot (k_1 + rk_1) - [NO_2^-]_0 \cdot k_1, \quad (15)$$

mit $k_1$, $k_4$, $K_{S,HNO2}$, $K_{S,H_3O_2^+}$ und $[H_3O^+]$ wie oben beschrieben.

$[H_2O_2]_0$ und $[NO_2^-]_0$ bezeichnen die initialen Anfangskonzentrationen zum Zeitpunkt des Vermischungsschrittes von $H_2O_2$ und $NO_2^-$ in der Wirklösung. Diese werden durch die Eduktkonzentrationen und die Art der Vermischung bzw. Verdünnung vorgegeben. Beispielsweise erhält man im Falle einer Eduktkonzentration von 200 mM $H_2O_2$ in Eduktlösung 1 und 200 mM $NO_2^-$ in Eduktlösung 2 und einem Vermischungsverhältnis von 1:1 initiale Konzentrationen von $[H_2O_2]_0 = [NO_2^-]_0 = 100$ mM.

Zudem ist

$$r=0{,}11, \quad (16)$$

wobei rein Ausgasungskoeffizient ist, der die Bildung von $NO_x$ aus $NO_2^-$ beschreibt und weiter unten näher beschrieben wird.

Exemplarisch sind in Fig. 23 die Wirksamkeitsbereiche, welches sich durch Ungleichung (5) bei einer Temperatur von 20 °C für gleiche initiale Konzentrationen von $[H_2O_2]_0 = [NO_2^-]_0$ ergeben, für alle Kombinationen von Einwirkzeiten $Z_E$ ausgewählt aus 15 s, 30 s, 50 s bzw. 90 s und Verarbeitungszeiten $Z_A$ ausgewählt aus 2s, 15s, 30s und 75, dargestellt. Diese Abbildung kann dem Fachmann zur Auslegung des Verfahrens bei Raumtemperatur dienen.

Da die Ausgangsstoffe ($NO_2^-$ und $H_2O_2$) mit der Zeit umgesetzt werden, nimmt die effektive Reaktionsrate der Reaktion zwischen $H_2O_2$ und $NO_2^-$ stetig ab. Aufgrund der kurzen Halbwertszeit der Reaktionsprodukte werden diese nicht akkumuliert und somit ist die momentane Reaktionsrate von $H_2O_2$ und $NO_2^-$ für die Wirksamkeit der Wirklösung zu einem gegebenen Zeitpunkt während der Einwirkzeit maßgeblich. Für den Einsatz der Wirklösung als Desinfektionsmittel ist es notwendig, dass die Wirksamkeit für eine definierte minimale Wirkdauer gegeben ist. Daher darf die zeitintegrierte Reaktionsrate W einen minimalen Wert nicht unterschreiten. Die heuristische Formel (5) erlaubt es, für Dekontaminationsanwendungen bei einer bestimmten Prozesstemperatur anwendbare Konzentrationen von $H_2O_2$ und $NO_2^-$ und einen entsprechenden pH-Wert zu wählen.

Im Gegensatz zu vegetativen Bakterien sind Bakteriensporen und unbehüllte Viren mit alkoholbasierten Mitteln nicht oder nur nach unzureichend langer Zeit inaktivierbar. Bei einer Reaktionsrate $W \geq 10$ mM werden nicht nur vegetative Bakterien, sondern auch Bakteriensporen inaktiviert.

In einer Ausführungsform ist die zeitintegrierte Reaktionsrate W der Reaktion zwischen $H_2O_2$ und $NO_2^-$ größer oder gleich 17.

Bei einer Reaktionsrate $W \geq 17$ mM werden neben vegetativen Bakterien und Bakteriensporen auch unbehüllte Viren inaktiviert.

In einer Ausführungsform, die sich ausschließlich auf vegetativen Bakterien richtet ist W=0,3, insbesondere 0,5.

Eine höhere zeitintegrierte Reaktionsrate W erhöht die desinfizierende Wirkung auf der mit Wirklösung kontaktierten Oberfläche.

Weiterhin spielen die Dauer des Verarbeitungszeitraums und des Einwirkzeitraums eine bedeutende Rolle bei der Effektivität des Desinfektionsverfahrens.

Der Verarbeitungszeitraum $Z_A$ umfasst den Vermischungsschritt und den Verteilungsschritt, wobei der Verteilungsschritt zeitgleich mit dem Vermischungsschritt zum Zeitpunkt *to=0* beginnen kann, oder dem nachgestellt sein kann. Der Verarbeitungszeitraum beginnt bei *to=0.*

Weiterhin können auch vor dem Verarbeitungszeitraum, also vor dem Zeitpunkt *to=0* relevante Schritte erfolgen, wie z.B. ein Verdünnungsschritt. Diese Schritte sind aber für das Zeitintervall zur Berechnung der zeitintegrierten Reaktionsrate nicht relevant und können daher vor *to=0* liegen.

Gegenstand der Erfindung ist, dass der Verarbeitungszeitraum, der zum Zeitpunkt $t_1$ endet, ausgewählt ist aus einem Bereich von $0 < t_1 \leq 75$ s, insbesondere ausgewählt ist aus dem Bereich $0 < t_1 \leq 30$ s, insbesondere ausgewählt ist aus einem Bereich $0 < t_1 \leq 15$s, insbesondere ausgewählt ist aus einem Bereich $0 < t_1 \leq 2$s.

In einigen Ausführungsformen beginnt die Einwirkzeit nach 2s.

In einigen Ausführungsformen beginnt die Einwirkzeit nach 15s.

In einigen Ausführungsformen beginnt die Einwirkzeit nach 30s.

In einigen Ausführungsformen beginnt die Einwirkzeit nach 75s.

In einigen Ausführungsformen ist ein längerer Verarbeitungszeitraum notwendig, der zum Zeitpunkt $t_1$ endet, wobei dieser ausgewählt ist aus einem Bereich von $15 < t_1 \leq 75$ s, insbesondere ausgewählt ist aus dem Bereich $30 < t_1 \leq 75$ s, insbesondere ausgewählt ist aus einem Bereich $50 < t_1 \leq 75$s.

In einigen Ausführungsformen ist ein kürzerer Verarbeitungszeitraum notwendig, der zum Zeitpunkt $t_1$ endet, wobei dieser ausgewählt ist aus einem Bereich von $0 < t_1 \leq 30$ s, insbesondere ausgewählt ist aus dem Bereich $0 < t_1 \leq 15$ s, insbesondere ausgewählt ist aus einem Bereich $0 < t_1 \leq 2$s.

In einigen Ausführungsformen ist ein Verarbeitungszeitraum notwendig, der zum Zeitpunkt $t_1$ endet, wobei dieser ausgewählt ist aus einem Bereich von $2 < t_1 \leq 75$ s, insbesondere ausgewählt ist aus dem Bereich $2 < t_1 \leq 30$ s, insbesondere ausgewählt ist aus einem Bereich $2 < t_1 \leq 15$s.

Der Verarbeitungszeitraum muss ausreichend groß sein, um jeden Punkt der zu desinfizierenden Oberfläche mit Wirklösung zu benetzen. Gleichzeitig muss der Verarbeitungszeitraum aber auch so klein gewählt sein, dass nach Verteilung der Wirklösung auf der zu desinfizierenden Oberfläche noch ausreichend reaktive Wirklösung vorhanden ist, um eine desinfizierende Wirkung zu erzielen.

Der Einwirkzeitraum, der zum Zeitpunkt $t_1$ beginnt, und zum Zeitpunkt $t_2$ endet, beträgt maximal 90 s. Ein kurzer Einwirkzeitraum ist vorteilhaft, um das Desinfektionsverfahren, insbesondere zur Oberflächendesinfektion, in möglichst kurzer Zeit durchführen zu können.

In einer weiteren Ausführung beträgt der Einwirkzeitraum, der zum Zeitpunkt $t_1$ beginnt, und zum Zeitpunkt $t_2$ endet, maximal 50 s, insbesondere maximal 30 s, und insbesondere maximal 15 s. Diese Ausführungsform ist insbesondere für die Desinfektion von Haut, wie beispielsweise Handdesinfektionen, geeignet.

Der Einwirkzeitraum muss ausreichend groß sein, um eine desinfizierende Wirkung zu erhalten.

Weiterhin sollte der Zeitbereich (Summe aus $Z_A$ und $Z_E$), insbesondere bei Anwendungen in einer Handdesinfektion ausreichend kurz gewählt sein, um die notwendige desinfizierende Wirkung in einem noch angemessenen Bereich zu erzielen. Eine zu lange Zeitspanne, wie beispielsweise über 10 Minuten, ist bei einer Händedesinfektion, selbst im klinischen Bereich, weder praktikabel noch sinnvoll anwendbar.

Die Vermischung der Edukte $H_2O_2$ und $NO_2^-$ zur Herstellung der Wirklösung kann vor Kontakt mit der zu desinfizierenden Oberfläche stattfinden, oder direkt auf der zu desinfizierenden Oberfläche erfolgen. Der Vermischungsschritt kann ohne äußere Einwirkung durch Diffusion und Konvektion erfolgen, durch mechanische Verteilung unterstützt werden, oder in einem Sprühprozess, bei dem die Edukte gemeinsam auf die zu desinfizierende Oberfläche gesprüht werden, integriert sein.

Des Weiteren spielt der *pH*-Wert eine entscheidende Rolle in dem erfindungsgemäßen Desinfektionsverfahren.

Der *pH*-Wert der Wirklösung auf der mit Wirklösung kontaktierten Oberfläche liegt im Bereich von $2{,}1 \leq pH \leq 6{,}8$, insbesondere in einem Bereich von $2{,}5 \leq pH \leq 5$, und insbesondere in einem Bereich von $3{,}3 \leq pH \leq 4{,}7$.

Die Reaktionsrate der Reaktion zwischen $H_2O_2$ und $NO_2^-$ hängt gemäß (6) vom *p*H-Wert der Lösung ab. Mit fallendem *pH*-Wert, also mit steigender Konzentration an $H_3O^+$, nimmt die Reaktionsrate $k_1$ zu. Bei niedrigen *pH*-Werten ist daher die desinfizierende Wirkung der Wirklösung höher, allerdings erlauben niedrige *pH*-Werte auf Grund der hohen Reaktionsrate von $H_2O_2$ und $NO_2^-$ in Kombination mit der Kurzlebigkeit der entstehenden Reaktionsprodukte keinen ausreichend großen Verarbeitungs- und Einwirkzeitraum. Bei höheren *pH*-Werten verringert sich die Reaktionsrate von $H_2O_2$ und $NO_2^-$ signifikant, wodurch allerdings auch die desinfizierende Wirkung der Wirklösung abnimmt.

Im Gegensatz zur Dekontamination in Suspensionen wurde festgestellt, dass eine Ansäuerung bei der Dekontamination von Oberflächen zu einer signifikanten Verschlechterung der Wirkung führen kann. Dies resultiert aus der Notwendigkeit, dass die Flüssigkeit auf der Oberfläche in einem Verteilungsschritt auf der Oberfläche aufgebracht und/oder verteilt werden muss und bei strukturierten und porösen Oberflächen durch Diffusion in die Oberfläche eindringen muss. Die Wirklösung darf ihre Desinfektionswirkung während dieser Zeit nicht verlieren, was durch einen zu niedrigen *pH*-Wert jedoch bewirkt wird. In diesem Fall werden die Edukte zu schnell abgebaut, bevor sie an jeder Stelle auf der Oberfläche ihre antimikrobielle Wirkung entfalten können. Dieses Problem wird durch die vorliegende Erfindung für eine Wirklösung aus mindestens $NO_2^-$ und $H_2O_2$ gelöst, indem ein *pH*-Wert-Bereich identifiziert wird, in welchem

der Einsatz als Mittel zur Oberflächendesinfektion möglich ist.

Viele Oberflächen haben selbst eine *pH*-Wert regulierende Eigenschaft, insbesondere eine Pufferwirkung, wie zum Beispiel die Hautoberfläche. Der *pH*-Wert, der für das Verfahren der vorliegenden Erfindung ausschlaggebend ist, ist deshalb der *pH*-Wert, der sich auf der mit Wirklösung benetzten Oberfläche ergibt. Dem Fachmann sind solche puffernden Oberflächen und ihre puffernde Wirkung bekannt. Nähere Details zur Pufferwirkung der Haut in diesem Desinfektionsverfahren sind in Fig. 4 dargestellt.

In einer Ausführungsform der vorliegenden Erfindung soll das Desinfektionsverfahren zur Desinfektion einer den *pH*-Wert stark puffernden Oberfläche, insbesondere Haut, oder anderen organischen Oberflächen, angewendet werden, wobei sich ein geeigneter *pH*-Wert auf der Oberfläche dadurch ergibt, dass der pH-Wert der Wirklösung vor Kontakt mit der zu desinfizierenden Oberfläche im Bereich von 2,1 bis 4,5, insbesondere in einem Bereich von 2,1 bis 3,6, insbesondere in einem Bereich von 2,1 bis 3,2 liegt.

Dieser *pH*-Wert wird durch den Kontakt mit der puffernden Oberfläche in Abhängigkeit der Oberflächenbeschaffenheit um 0,2 bis 1,7, insbesondere um 0,2 bis 0,8 angehoben.

Dadurch ist der *pH*-Wert der Wirklösung geringer als der *pH*-Wert der Wirklösung auf der zu desinfizierenden Oberfläche. Durch diese Eigenschaft reagieren die Edukte außerhalb der zu desinfizierenden Oberfläche schnell ab und akkumulieren nicht in der Umwelt. Auf der zu desinfizierenden Oberfläche, insbesondere bei der Oberfläche eines Körperteils, insbesondere einer Hand, hingegen läuft die Reaktion zwischen $H_2O_2$ und $NO_2^-$ durch die Pufferwirkung der Oberfläche etwas langsamer ab, wodurch sich die desinfizierende Wirkung entfalten kann. Somit bleibt die desinfizierende Wirkung auf der bestimmungsgemäßen Oberfläche ausreichend lang wirksam, auf nicht bestimmungemäßen Oberflächen erfolgt ein schneller Abbau der Edukte und somit keine Akkumulation.

In einer Ausführungsform sind die Ausgangsstoffmengen der Edukte identisch, insbesondere bei Anwendungen wenn Ausgasen von $NO_x$ vernachlässigbar ist, somit werden $NO_2^-$ und $H_2O_2$ vollständig umgesetzt. Dadurch werden keine bioziden Wirkstoffe in die Umwelt abgegeben.

In einer Ausführungsform beträgt die Effizienz $E = W/W_{max}$ des Verfahrens bei Prozessbedingt vorgegebenen Zeiten $t_1$ und $t_2$ mindestens 10 %, insbesondere mindestens 20 %, insbesondere mindestens 30 %, wobei

$$W_{\max} = \min([\mathrm{H_2O_2}]_0, [\mathrm{NO_2^-}]_0)\ (\exp(-Gt_1) - \exp(-Gt_2)) \qquad (17)$$

mit $G = \ln\left(\frac{t_2}{t_1}\right)/(t_2 - t_1)$ den $t_1$ maximal erreichbaren Wirksamkeitsparameter bezeichnet und $\min([\mathrm{H_2O_2}]_0, [\mathrm{NO_2^-}]_0)$ die minimale Konzentration ausgewählt aus den initialen Konzentrationen $[H_2O_2]_0$ und $[\mathrm{NO_2^-}]_0$ bezeichnet. Dies stellt sicher, dass die verwendeten Edukte durch sinnvolle Wahl der wählbaren Prozessparameter pH-Wert, Temperatur und Ausgangskonzentrationen der Edukte effizient eingesetzt werden. Exemplarisch sind Prozessparameter mit unterschiedlicher Effizienz in Fig. 23 für einen Prozess mit $t_1$=15 s und $t_2$ = 45 s dargestellt. Hierbei wurden als Variable die initialen Konzentrationen $[H_2O_2]_0$ und $[NO_2^-]_0$ sowie der pH-Wert variiert.

In einer Ausführungsform unterscheiden sich die Ausgangsstoffmengen an Edukten um weniger als 10 % voneinander, insbesondere die Ausgangsstoffmenge von $NO_2^-$ ist um 2 % bis 10 % höher als die Ausgangsstoffmenge von $H_2O_2$. Der genaue Wert ist für eine gegebene Anwendung, also für eine gegebene Oberfläche und Flüssigkeitsmenge, zu bestimmen. In diesem Fall werden als stabile Endprodukte ausschließlich Nitrat und Wasser gebildet, während $NO_2^-$ und $H_2O_2$ vollständig umgesetzt werden. Dadurch werden keine bioziden Wirkstoffe in die Umwelt abgegeben. Das erfindungsgemäße Desinfektionsverfahren ist somit besonders umweltfreundlich.

Eine geringfügig höhere Ausgangsstoffmenge an $NO_2^-$ im Vergleich zu $H_2O_2$ ist hilfreich, um den Verlust an wirksamem $NO_2^-$ durch Ausgasen von $NO_x$ vorzubeugen.

Das Ausgasen von NOx ist bei einer Oberflächendesinfektion auf Grund der größeren Oberfläche deutlich stärker als bei einer Desinfektion, die in Lösung oder Suspension stattfindet. Beim Verteilen der Wirklösung auf einer Oberfläche entsteht nur ein dünner Flüssigkeitsfilm, bei dem große Anteile des eingesetzten $NO_2^-$ als gasförmige Stickoxide ($NO_x$) freigesetzt werden können. Dies hat unter anderem zur Folge, dass bis zu 10% des in die Flüssigkeit eingebrachten $NO_2^-$ in Form von NO(g) oder insbesondere $NO_2$(g) ausgasen. Die Ausgasung führt zu einem beschleunigten Abbau von $NO_2^-$ in einer Wirklösung aus $H_2O_2$ und $NO_2^-$ auf Oberflächen, verglichen mit einer identisch angesetzten Wirklösung in Suspension. Die Ausgasung beeinflusst die Reaktionskinetik und sollte auch aus gesundheitlichen Gründen niedrig gehalten werden.

Die $NO_x$-Emissionen sind auf zwei grundlegende Prozesse zurückzuführen: Einerseits kann der Einsatz einer Säure zur Einstellung des *pH*-Wertes direkt eine $NO_x$-Ausgasung bewirken, so beispielsweise der in Fig. 1 dargestellte

Prozess:

$$HNO_2 + HNO_2 \rightarrow NO + NO_2 + H_2O \rightarrow NO(g) + NO_2(g) + H_2O. \qquad (18)$$

Für den Prozess (18) ist die Anwesenheit von $H_2O_2$ nicht erforderlich. Andererseits kann die Bildung von ONOOH, welche die Anwesenheit von $H_2O_2$ erfordert, durch die Reaktion (19)

$$ONOOH \rightarrow NO_2 + OH \qquad (19)$$

durch anschließende Ausgasung von $NO_2$ zu den $NO_x$-Emissionen beitragen.

Die Ausgasung von $NO_x$ wurde mittels Experimenten und Computersimulationen untersucht. In einer Ausführung der vorliegenden Erfindung wird die Ausgasung als Ausgasungsrate durch die Formel

$$R_{degas} = R_1 \times r \qquad (20)$$

beschrieben, welche proportional zur effektiven Vernichtungssrate $R_1$ von $NO_2^-$ und $H_2O_2$ durch Reaktion (1) angenommen werden kann, und zur Vernichtung von $NO_2^-$ entsprechend der Formel

$$\frac{d[NO_2^-]}{dt} = -R_1 - R_{degas} = -k_1[H_2O_2][NO_2^-] \times (1 + r) \qquad (21)$$

beiträgt. Hierbei bezeichnet r einen auf $R_1$ bezogenen Anteil, welcher zur Ausgasung von $NO_2^-$ in Form von $NO_x$ führt. Diese Form resultiert daraus, dass die Reaktion

$$ONOOH \rightarrow NO_2 + OH \qquad (22)$$

den wesentlichen Beitrag zur Ausgasung bei der Oberflächendekontamination liefert. Während *r* in Suspensionsversuchen üblicherweise vernachlässigbar klein, etwa im Bereich von $r \leq 0.01$ ist, kann *r* bei der Oberflächendekontamination Werte im Bereich $r \leq 0.11$ annehmen. Der konkrete Wert hängt von der jeweiligen Anwendung, insbesondere von der Schichtdicke des Flüssigkeitsfilms, ab. Die auftretende Ausgasung beeinflusst also bei einer Oberflächendesinfektion die Reaktionskinetik der Reaktion von $H_2O_2$ und $NO_2^-$, was bei einer Desinfektion in Suspension zu vernachlässigen wäre.

Eine Möglichkeit, die Menge an ausgasenden $NO_x$, zu begrenzen, ist die Ausgangskonzentration von $NO_2^-$ zu begrenzen.

Im erfindungsgemäßen Desinfektionsverfahren soll daher eine maximale Ausgangskonzentration an $NO_2^-$ zum Zeitpunkt $t_0$ eine Konzentration von 300 mM, insbesondere von 200 mM, insbesondere von 100 mM nicht überschritten werden.

Dadurch wird die Menge an ausgegasten $NO_x$ so gering wie möglich gehalten, und gesundheitsschädliche Nebenwirkungen des Desinfektionsverfahrens werden somit auf ein Mindestmaß limitiert.

Das erfindungsgemäße Desinfektionsverfahren kann zur Desinfektion von Oberflächen eingesetzt werden. Das erfindungsgemäße Desinfektionsverfahren kann insbesondere zur Desinfektion von Haut verwendet werden

Das Desinfektionsverfahren der vorliegenden Erfindung kann weiterhin eingesetzt werden zur Dekontamination von Medizinprodukten, insbesondere von thermolabilen Medizinprodukten wie Schläuche von Endoskopen sowie von Behältern und Wannen.

Das Desinfektionsverfahren der vorliegenden Erfindung kann weiterhin eingesetzt werden zur Dekontamination von Saatgut, Nutzpflanzen, tierischen Produkten, Lebensmitteln, Verpackungen sowie von Getränkebehältern oder Getränkeleitungen.

In einer Ausführungsform des erfindungsgemäßen Desinfektionsverfahrens können den Edukten und/oder der Wirklösung Säurepuffer bzw. Säurepufferlösungen zugesetzt werden. Als Puffer können beispielsweise Citratpuffer, Essigsäure-Acetat-Puffer, Phosphat-CitratPuffer, Phosphat-Puffer oder Citratpuffer eingesetzt werden. Citrathaltige Pufferlösungen eignen sich insbesondere aufgrund ihres angenehmen Geruchs.

In einer Ausführungsform des erfindungsgemäßen Desinfektionsverfahrens können den Edukten vor und/oder während des Vermischungsschrittes Zusatzstoffe hinzugefügt werden. Denkbare Zusatzstoffe umfassen unter anderem Lösungsmittel, Basen, Duftstoffe, Farbstoffe und/oder weitere Desinfektionsmittel, und/oder Ozon.

Des Weiteren können Suspensionen mit nicht-wasserlöslichen Stoffen erzeugt werden, insbesondere durch Beimischungen von Fetten und Tensiden.

In einer weiteren Ausführungsform können eine oder mehrere Plasmaquellen genutzt werden, um eines oder mehrere der Edukte herzustellen.

Somit wäre es möglich, die Edukte $H_2O_2$ und $NO_2^-$ aus Luft und Wasser mittels Strom herzustellen. Im Stand der Technik sind Plasmaverfahren ausreichend offenbart, so dass der Fachmann ein entsprechendes Plasma auswählen kann (siehe auch Lukes et al., 2015, EP17150571.2).

In einer weiteren Ausführung der vorliegenden Erfindung wird durch das Plasma zusätzlich Ozon hergestellt, welches Teil der Wirklösung sein kann.

Das Verfahren zur Desinfektion von Oberflächen, das Gegenstand dieser Erfindung ist, zeichnet sich dadurch aus, dass es eine sporizide Wirkung hat. Es gibt in Deutschland kein zugelassenes Desinfektionsmittel zur Desinfektion von Haut, dass auch gegenüber Bakteriensporen eine desinfizierende Wirkung hat. Weiterhin ist das erfindungsgemäße Verfahren, geruchsarm und ist vorteilhaft gegenüber üblichen Desinfektionsverfahren zur Desinfektion von Haut, z.B. Alkohol, weil es die Haut nicht austrocknet.

Weiterhin Gegenstand der vorliegenden Offenbarung ist eine Vorrichtung zur Bereitstellung und/oder Applikation der (in situ gebildeten) Desinfektionslösung für das genannte Desinfektionsverfahren, bestehend aus

- einen Speicherbereich, umfassend mindestens einen Speicher in dem mindestens ein Edukt vorgehalten wird,
- eine Vorrichtung zur Vermischung der Edukte und Bereitstellung der Wirklösung,
- eine Abgabevorrichtung der Wirklösung auf die zu desinfizierende Oberfläche,
  oder
- einem Speicherbereich, umfassend mindestens einen Speicher in dem mindestens ein Edukt vorgehalten wird,
- eine Vorrichtung zur Abgabe der Edukte auf die zu desinfizierende Oberfläche, wobei die Abgabevorrichtung derart ausgestaltet ist, dass eine Vermischung der Edukte der Wirklösung auf oder vor der zu desinfizierenden Oberfläche erfolgt.

Der Speicherbereich und die Vermischungsvorrichtung sowie die Abgabevorrichtung sind fluidisch miteinander verbunden, so dass die Edukte durch schließbare Öffnungen nach Aktivierung vom Speicherbereich in die Vermischungsvorrichtung und von dort als Wirklösung über die Abgabevorrichtung auf die zu desinfizierende Oberfläche gelangen können. Alternativ kann die Vermischung der Edukte auch außerhalb der Vorrichtung mittels beispielsweise zweier aufeinander gerichteter Düsen erfolgen.

Die Edukte (mindestens $H_2O_2$, $NO_2^-$, und Säure) können auf verschiedene Weisen vorgehalten und vermischt werden. In einer Ausführung der vorliegenden Offenbarung werden $H_2O_2$, $NO_2^-$ und eine Säure jeweils getrennt, also in drei getrennten Speichern vorgehalten.

In einer weiteren Ausführung der vorliegenden Offenbarung werden $H_2O_2$ und die Säure zusammen in einem Speicher, und $NO_2^-$ in einem zweiten getrennten Speicher vorgehalten. In dieser Ausführung werden nur zwei Speicher zur Vorhaltung der Edukte benötigt (Fig. 2).

In einer weiteren Ausführung der vorliegenden Offenbarung wird $NO_2^-$ zusammen mit der Säure, insbesondere mit Salpetersäure, in einem Speicher vorgehalten, und $H_2O_2$ wird in einem zweiten Speicher vorgehalten. Durch Ansäuerung von $NO_2^-$ mit Salpetersäure entstehen $NO_x$-Gase oberhalb der Flüssigkeit, welche zu Drücken von mehreren Bar in dem jeweiligen Speicher führen. Dieser Druck kann in einer weiteren Ausführung der vorliegenden Offenbarung als Antrieb für die Vorrichtung genutzt werden.

In einer weiteren Ausführung der vorliegenden Offenbarung werden sowohl $H_2O_2$ als auch $NO_2^-$ angesäuert vorgehalten. In dieser Ausführungsform können die Ansäuerung zum Druckaufbau und die Ansäuerung, die für die Reaktion von $H_2O_2$ und $NO_2^-$ (1) nötig ist, unabhängig voneinander durchgeführt werden. Es ist möglich, die Edukte in verschiedenen Formen vorzulegen.

In einer Ausführung der vorliegenden Offenbarung ■ können die Edukte in den für die Wirksamkeit des Produktes benötigten Konzentrationen vorgelegt werden.

In einer weiteren Ausführung der vorliegenden Offenbarung kann die Vorrichtung einen Verdünnungsbereich umfassen, in dem die Edukte mit Lösungsmitteln und/oder Zusatzstoffen verdünnt werden. Die Edukte können in dieser Ausführungsform als Konzentrat vorgelegt werden, welches dann im Prozess verdünnt wird. Wie oben beschrieben, kann als Lösungsmittel für das beschriebene Verfahren Wasser verwendet werden..

Denkbare Zusatzstoffe umfassen unter anderem, Basen, Pufferlösungen, Duftstoffe, Farbstoffe und/oder Ozon. In dieser Ausführungsform können durch die Vorlage des Konzentrates und anschließende Verdünnung 3 bis 200 Teile Wirklösung aus einem Teil Konzentrat gewonnen werden.

In einer weiteren Ausführung der vorliegenden Offenbarung können die Edukte als Salze vorgelegt werden, welche im Prozess in Lösung gebracht werden. $H_2O_2$ liegt insbesondere als Salz, insbesondere als Natriumpercarbonat, oder in Lösung vor, während $NO_2^-$ insbesondere als Salz, insbesondere als Natrium oder Kaliumsalz, oder in Lösung vorliegt. Die Edukte können in unterschiedlicher Form vorliegen.

Ebenfalls ist es möglich $H_2O_2$ in höheren Konzentrationen zuzusetzen, insbesondere in Konzentrationen größer gleich 0.5 % (v/v), sodass $H_2O_2$ selbst eine bakterizide und/oder viruzide, jedoch keine sporizide Wirkung besitzt. Die sporizide Wirkung des erfindungsgemäß durchgeführten Verfahrens wird dann weiterhin durch die Ungleichung (5)

beschrieben. Es ist möglich, die Edukte, gemischt mit Zusatzstoffen oder in Reinform, in einer entsprechenden Vorrichtung bereit zu halten.

Bei Aktivierung werden die Edukte und/oder Zusatzstoffe aus den Speichern abgegeben. Durch Vermischung der Edukte und/oder Zusatzstoffe wird die Wirklösung gebildet.

Weiterhin können die Speicher über ein Auslassventil verfügen, durch welches Flüssigkeit abgelassen und verworfen werden kann.

In einer weiteren Ausführung der vorliegenden Offenbarung sind die Speicher mit Füllstandssensoren ausgestattet, insbesondere mit mechanischen Füllstandssensoren oder elektrischen, insbesondere kapazitiven, induktiven Füllstandssensoren, optischen Füllstandssensoren oder Ultraschall-Füllstandssensoren, kombiniert mit einer Abschaltvorrichtung, welche die Aktivierung des Gerätes verhindert, falls ein Minimalfüllstand eines Eduktes unterschritten wird.

Zusätzlich können die wechselbaren Speicher mit einer Vorrichtung versehen sein, die die eindeutige Identifizierung des Inhalts erlaubt und bei falscher Befüllung die Aktivierung des Gerätes verhindert.

In einer weiteren Ausführung der vorliegenden Offenbarung kann eine Prozesskontrolle am Speicherbereich und/oder zwischen Speicherbereich und Abgabevorrichtung durch ein oder mehrere Barometer und/oder einen oder mehrere Durchflusssensoren stattfinden, insbesondere kombiniert mit einer Abschaltvorrichtung, welche die Aktivierung des Gerätes verhindern kann, falls der geforderte Betriebsdruck oder der Betriebsdurchfluss aufgrund von Fehlfunktion oder leeren Vorratsspeicher nicht eingehalten wird.

In einer weiteren Ausführung der vorliegenden Offenbarung verfügt die Vorrichtung über Sensoren, um die Konzentration der Edukte oder der Zusatzstoffe zu bestimmen. Bei den Sensoren kann es sich insbesondere um Leitfähigkeits-, Kapazitäts- oder pH-Elektroden handeln. Vorteilhaft für die Prozesskontrolle ist weiterhin die Nutzung von Absorptionsspektroskopie im ultravioletten und sichtbaren Bereich zur Bestimmung der Eduktkonzentrationen und/oder des pH-Wertes.

Insbesondere umfasst die Vorrichtung einen Sensor oder eine Kamera, die dazu dienen, die abgegebene Flüssigkeitsmenge für den jeweiligen Verwender passend zu dosieren, wobei die Steuerung der Abgabe insbesondere durch Erkennung der Größe der zu desinfizierenden Oberfläche erfolgen kann. Weiterhin kann die Kamera und/oder ein Sensor zur Nutzer- bzw. Nutzungskontrolle verwendet werden. Die Vorrichtung kann zur weiteren Auswertung dieser Daten einen Mikroprozessor enthalten und/oder eine Schnittstelle für eine externe Datenverarbeitung, insbesondere eine drahtlose Datenschnittstelle. In einer weiteren Ausführung umfasst die Vorrichtung einen oder mehrere Temperatursensoren und/oder Heiz- oder Kühlelemente, insbesondere Peltier-Elemente, um eine gleichbleibende Temperatur der Ausgangsflüssigkeiten zu gewährleisten und/oder die Flüssigkeit für auf eine für den Nutzer angenehme Temperatur zu temperieren.

In einer weiteren Ausführung der vorliegenden Offenbarung umfasst die Vorrichtung eine oder mehrere Plasmaquellen, welche mindestens eines der Edukte erzeugt, wobei die Plasmaquelle in den mindestens einen Speicher integriert ist, oder dem mindestens einen Speicher vorgeschaltet ist, oder der Vorrichtung zur Vermischung der Edukte vorgeschaltet ist oder in die Vorrichtung zur Abgabe der Edukte integriert ist. Die Plasmaquellen können dazu dienen, $H_2O_2$ und/oder $NO_2^-$ herzustellen sowie eine ausreichende Ansäuerung der jeweiligen Flüssigkeiten zu bewirken. Hierbei kann insbesondere eine heiße Plasmaquelle, insbesondere ein Lichtbogen oder eine Mikrowellenentladung zur Anreicherung von $NO_2^-$ verwendet werden, sowie eine kalte Plasmaquelle, insbesondere eine dielektrisch behinderte Entladung, eine Corona-Entladung oder eine Radiofrequenz-Entladung zur Anreicherung von $H_2O_2$.

Weiterhin können die Speicher über einen Wassereinlass verfügen, über welchen Wasser hinzugefügt werden kann, insbesondere von einem Hausanschluss. Insbesondere kann die Vorrichtung einem handelsüblichen Wasserhahn oder Wasserspender vorgeschaltet sein.

In einer weiteren Ausführung der vorliegenden Offenbarung werden die Edukte im Vermischungsbereich zusätzlich verdünnt, und/oder mit Zusatzstoffen angereichert. In einer besonderen Ausführung der vorliegenden Offenbarung findet die Verdünnung der Edukte und/oder die Anreicherung mit Zusatzstoffen in einem dem Vermischungsbereich vorgelagerten Verdünnungsbereich statt.

In einer weiteren Ausführung der vorliegenden Offenbarung kann der Vermischungsbereich eine Mischkammer oder Mischstrecke sein, in welcher die Flüssigkeiten vermischt werden. Hierbei kann die Mischstrecke als mikrofluidischer Mischer ausgeführt sein.

In einer weiteren Ausführung der vorliegenden Offenbarung verfügt die Vorrichtung über eine Wasserzuleitung, welche aus einer weiteren Vorratskammer oder einem externen Wasseranschluss, insbesondere einer Hauswasserleitung gespeist wird. Durch Öffnung eines Ventils oder durch Betrieb einer Pumpe wird das Wasser zur Verdünnung der Wirklösung auf die gewünschte Konzentration hinzugefügt. Hierbei sind die Zuleitungen des Vermischungsbereichs vorzugsweise so ausgeführt, dass die konzentrierten $NO_2^-$ und $H_2O_2$-Lösungen mit Wasser verdünnt werden, bevor sie in Kontakt kommen.

In einer besonderen Ausführung der vorliegenden Offenbarung sind der Vermischungsbereich und der Austrittsbereich identisch, sodass die Vermischung der Edukte beim Austritt aus der Vorrichtung und/oder auf der zu desinfizierenden Oberfläche stattfindet.

Weiterhin verfügt die Vorrichtung über eine Druckluftzuleitung welche zum Verteilen der Wirklösung/des Wirkaerosols eingesetzt wird, wobei die Druckluft durch eine interne Pumpe bzw. einen Kompressor bereitgestellt wird oder extern bereitgestellt wird.

Weiterhin verfügt die Vorrichtung über eine Stromversorgung, insbesondere über eine interne Batterie, einen Akku, eine Solar- oder Brennstoffzelle.

In einer weiteren Ausführung der vorliegenden Offenbarung ist die Vorrichtung mit einem Applikator ausgestattet, welcher zur Vermischung und/oder Verteilung der Flüssigkeiten dient. Der Applikator kann aus einem Aerosolgenerator bestehen, welcher ein Aerosol erzeugt, welches durch einen Luftstrom verteilt wird, wobei es sich bei dem Aerosolgenerator um einen oder mehrere Ultraschallvernebler handeln kann oder um einen oder mehrere Jet-Vernebler handeln kann.

In einer weiteren Ausführung der vorliegenden Offenbarung kann der Applikator aus Flüssigkeitspumpen bestehen, welche die Flüssigkeiten fördern, wobei es sich insbesondere um Handpumpen, Kolbenpumpen, Membranpumpen, peristaltische Pumpen, oder Venturi-Pumpen handeln kann, und wobei der Auslass des Applikators als Düse ausgeführt sein kann, welche die Flüssigkeit als Strahl oder Spray abgibt. Insbesondere können in einer weiteren Ausführungsform mindestens zwei Flüssigkeiten mit einer einzigen Pumpe gefördert und vor oder nach der Pumpe in einer Vermischeinheit vermischt.

In einer weiteren Ausführung der vorliegenden Offenbarung sind die Pumpen so ausgelegt, dass alle Pumpen durch einen gemeinsamen Antrieb betrieben werden. Bei dem Antrieb kann es sich um eine gemeinsame Welle bei peristaltischen Pumpen oder Kolbenpumpen handeln, oder um eine gemeinsame Druckluftversorgung im Falle von pneumatischen Pumpen oder Venturi-Pumpen.

In einer weiteren Ausführung der vorliegenden Offenbarung kann die Vorrichtung eine Wasseraufbereitungsanlage, insbesondere eine Destille, einen Osmosefilter oder einen Kohlefilter umfassen. Diese Wasseraufbereitungsanlage kann insbesondere dazu verwendet werden, Wasser aus einer Hauswasserleitung zu reinigen und eine definierte, insbesondere niedrigere Pufferkapazität zu erreichen. Dies ist für den Prozess vorteilhaft, da der pH-Wert für den Prozess ansonsten durch den Einsatz eines stärkeren pH-Puffers eingestellt werden muss.

Die Wirklösung kann als Aerosol, Flüssigkeitsstrahl oder Dampf auf die zu desinfizierende Oberfläche gebracht werden.

Figurenbeschreibung

Fig. 1: Darstellung des Reaktionsschemas der Reaktion zwischen $H_2O_2$ und $NO_2^-$.

Fig. 2: Schematische Darstellung des Desinfektionsverfahrens, bei dem die Edukte aus den Vorratsspeichern (1, 2) mit einem Lösungsmittel, gelagert in einem weiteren Vorratsspeicher (3) zum Erhalt der Wirklösung vermischt werden.

Fig. 3: Schematische Darstellung des Desinfektionsverfahrens, bei dem die Edukte aus den Vorratsspeichern (1,2) getrieben werden und mit einem Lösungsmittel, das in einem dritten Vorratsspeicher vorgehalten wird (3) in einem Vermischungsbereich (10) zum Erhalt der Wirklösung vermischt werden. Durch Zufuhr von Luft (18) wird die Wirklösung aus dem Vermischungsbereich ausgetrieben und auf die zu desinfizierende Oberfläche gegeben.

Fig. 4: pH-Werte, gemessen auf der Hand vor (links) und nach der Handdesinfektion (rechts) gemäß EN 1500 mit drei verschiedenen Wirklösungen und in den Wirklösungen vor der Handdesinfektion (mitte). Die Wirklösungen bestanden aus Ausgangsflüssigkeit A (50 mM $H_2O_2$ und der angegebenen Menge Citronensäure in %(w/v)), sowie Ausgangsflüssigkeit B (50 mM $NaNO_2$).

Fig. 5: Inaktivierung von E. Coli in Suspension mit drei verschiedenen Wirklösungen (Isopropanol als alleiniger Wirkstoff, Wirklösung mit 2% Citronensäure und 10% Citronensäure. Aufgetragen ist für x der Anteil an Desinfektionsmittel in % und für y die koloniebildenden Einheiten.

Fig. 6: Abhängigkeit der Wirksamkeit von der zeitintegrierten Reaktionsrate W gegenüber E. coli Bakterien. Für x ist die Konzentration an $NO_2^-$ und $H_2O_2$ in mmol zum Zeitpunkt $t_0=0$ aufgetragen, für y die Einwirkzeit in Sekunden. Für W>0.5 mM wird in allen Experimenten eine Reduktion von mindestens 3.2 $log_{10}$-Stufen erreicht. Für W<0.5 mM wird in allen Experimenten eine Reduktion von weniger als 3.2 $log_{10}$-Stufen erreicht.

Fig. 7: Wirksamkeitskurven mit konstantem Wirksamkeitsparameter W in mM (aufgetragen ist für x der pH-Wert

und für y die Anfangskonzentration an $H_2O_2$ bzw. $NO_2^-$ in mM bei gleicher Anfangskonzentration, Werte für $t_0$=0) sowie experimentell bestimmte $log_{10}$-Reduktion von Bakterien (E. Coli) in Suspension, dargestellt durch unterschiedlich große Punkte.

Fig. 8: Wirksamkeitskurven für die Oberflächendesinfektion ($t_1$=30 s, $t_2$=45 s) unter Berücksichtigung der $NO_x$ Ausgasung. Die Pfeile geben Start- und End-pH-Werte in den Handdesinfektionsversuchen an. Die Anfangskonzentration von $H_2O_2$ und $NO_2^-$ ist jeweils gleich (wie angegeben) gewählt. x-Achse: pH-Wert, y-Achse: Anfangskonzentration an $H_2O_2$ bzw. $NO_2^-$ zum Zeitpunkt $t_0$=0. A: 10% Citronensäure, B: 2% Citronensäure, C: 0,5% Citronensäure.

Fig. 9: Effekt der unterschiedlichen Ausgasung in Suspension und auf Oberflächen auf die Flüssigkeitschemie. Versuchsaufbau (links) mit Zufuhr von Luft (18) und gemessener Konzentrationsverlauf in einer Wirklösung mit 150 mM $NO_2^-$ und 150 mM $H_2O_2$ bei pH 3 bzw.

und pH 4 (rechts). Aufgetragen sind für x die Zeit in Sekunden und für y die Anzahl der detektierten $NO_x$ Moleküle pro Sekunde ($\times_{10}{}^{16}$).

Fig. 10: Effekt der unterschiedlichen Ausgasung in Suspension und auf Oberflächen auf die Flüssigkeitschemie. Versuchsaufbau (links) und berechneter Konzentrationsverlauf (rechts), mit x Zeit in Sekunden und y Konzentration in mM.

Fig. 11: Schematische Darstellung einer Vorrichtung mit 2 Vorratsspeichern und einer Verdünnungsflüssigkeit.

Fig. 12: Schematische Darstellung einer Vorrichtung, welche die Wirklösung als Aerosol bereitstellt.

Fig. 13: Schematische Darstellung einer Vorrichtung, welche die Wirklösung als Flüssigkeitsspray bereitstellt.

Fig. 14: Schematische Darstellung einer Vorrichtung, bei der die Wirklösung über einen Wasserhahn appliziert wird.

Fig. 15: Schematische Darstellung einer Vorrichtung, die in einer Wasserleitung zwischengeschaltet ist, welche zu einem Wasserhahn führt.

Fig. 16: Schematische Darstellung einer Vorrichtung, die eine Plasmaquelle beinhaltet, welche einen Teil der Edukte erzeugt.

Fig. 17: Arrhenius-Graph für die Reaktion von $NaNO_2$ und $H_2O_2$ mit einer Ausgangskonzentration von jeweils 5 mM unter Verwendung eines Citronensäure-Phosphat-Puffers bei pH 3 zwischen 0°C und 40°C.

Fig. 18: Gemessene Absorbanz und zugehörige Fits der Reaktionsprodukte $NO_3^-$, $H_2O_2$, $NO_2^-$ und $HNO_2$ aus der Reaktion von 73.2 mM $NaNO_2$, 58.9 mM $H_2O_2$ und 100 mM HCl bei 0 °C nach 5 min Reaktionszeit.

Fig. 19: Zeitaufgelöste Messung der Dichten von $HNO_2/NO_2$ (Gesamtdichte von $HNO_2$ und $NO_2^-$), $NO_3^-$ und ONOOH bei der Reaktion von 73.2 mM $NaNO_2$, 58.9 mM $H_2O_2$ und 100 mM HCl bei 0 °C.

Fig. 20: Absorbanz und Fit zum Zeitpunkt t=20 s bei der Reaktion von 73.2 mM $NaNO_2$, 58.9 mM $H_2O_2$ und 100 mM HCl bei 0 °C.

Fig. 21: Reduktionsfaktoren für unterschiedliche Arten von Mikroorganismen in Abhängigkeit vom Wirksamkeitsparameter W. Die horizontale Linie entspricht einer Reduktion der Mikroorganismenkonzentration um 99%.

Fig. 22: Wirksamkeitsbereiche bei einer Temperatur von 20 ° C und Einwirkzeiten $Z_E$ von 15 s, 30 s, 50 s bzw. 90 s (von oben nach unten). In den Bereichen oberhalb der Kurven gilt bei der jeweils angegebenen Dauer des Verarbeitungszeitraumes $Z_A$ ausgewählt aus 2s, 15s, 30s und 75 für den Wirksamkeitsparameter W >10mM. Aufgetragen ist für x der pH Wert und für y die initialen Konzentrationen $[H_2O_2]_0 = [NO_2^-]_0$ in mM.

Fig. 23: Wirksamkeitsbereich und Effizienz bei einer Temperatur von 20 ° C, einem Verarbeitungszeit von $Z_A$ = 15 s und einer Einwirkzeit $Z_E$ = 30 s. Der Bereich Oberhalb der mit W=10 mM markierten Kurve gibt den Bereich an, in welchem W >10mM gilt. Die mit Prozentzahlen markierten Kurven definieren jeweils Bereiche in denen die Effizienz mindestens den angegebenen Wert annimmt. Der schraffierte Bereich gibt exemplarisch jene Werte an, in denen

W>10 mM und E>45 % gilt. Aufgetragen ist für x der pH Wert und für y die initialen Konzentrationen $[H_2O_2]_0 = [NO_2^-]_0$ in mM.

In Fig. 11 ist eine entsprechende Konstruktion gezeigt, bei welcher die Kolbenpumpen (6, 7, 8) zum Fördern der Fluide über eine Kurbelwelle (12) angetrieben werden. Dabei werden die Kolben aufgezogen und entleert. Zunächst wird die Verdünnungslösung C durch Aufziehen des Kolbens (8) aus dem Vorratsspeicher C (3) in den Kolben gefördert. Über eine Fortführung der Kurbelwellendrehbewegung gelangt die Verdünnungslösung C in die Vermischungsvorrichtung (10). Dabei verhindern Rückschlagventile (5) das Zurückfließen der Verdünnungslösung C in den Vorratsspeicher C (3). Durch eine Phasenverschiebung von 90° auf der Kurbelwelle (12) erfolgt das Aufziehen und das anschließende Ausstoßen der Eduktlösungen A und B in die Vermischungsvorrichtung (10) zeitverzögert zu der Bereitstellung der Verdünnungslösung C. Somit ist gewährleistet, dass die Vermischung der Edukte A und B unter verdünnten Bedingungen stattfindet. Das ist wichtig, da die Reaktion von $H_2O_2$ und $NO_2^-$ nach Gleichung (5) von der Konzentration der Edukte abhängt und im konzentrierten Fall zu schnell ablaufen würde. Das Volumen der Vermischungsvorrichtung (10) ist so gewählt, dass die Summe der Einzelvolumina der Lösungen A, B und C aufgenommen werden können. In der Vermischungsvorrichtung (10) liegt nach der Zugabe der Eduktlösungen A und B zur Verdünnungslösung C die Wirklösung vor. Mittels des vierten Kolbens (9), welcher Luft über die Zufuhr 18 ansaugt, komprimiert und in Richtung Vermischungsvorrichtung ausstößt wird die Wirklösung aus der Vermischungsvorrichtung in die Abgabevorrichtung (11) (z.B. eine Düse) gepresst und anschließend auf der zu desinfizierenden Oberfläche appliziert.

Der Vorgang der Eduktvermischung und der anschließenden Abgabe des Wirkstoffs kann durch die Kurbelwellenkonstruktion kontinuierlich wiederholt werden. Die Ausstoßfrequenz der Wirklösung lässt sich über die Rotationsgeschwindigkeit der Kurbelwelle (12) einstellen.

Mittels eines optischen Sensors (17) wird berührungslos festgestellt ob sich eine zu desinfizierende Oberfläche im Sprühbereich befindet und welche Größe diese Fläche aufweist. Diese Information wird von einer Steuereinheit (14) ausgewertet und anschließend das Gerät aktiviert. Je nach Größe der zu desinfizierenden Oberfläche werden ein oder mehrere Sprühstöße abgegeben. In einer weiteren Ausführungsform wird mittels des optischen Sensors (17) eine Identifikation der Oberfläche und/oder des Benutzers vorgenommen. Dadurch kann im Rahmen einer nachgeschalteten Auswertung festgestellt werden, welche Oberflächen und/oder welche Benutzer wie oft und zu welcher Zeit welche Wirkstoffmenge erhalten haben.

Über eine Anzeigeeinheit (16) werden dem Anwender Informationen zum System und/oder zur desinfizierenden Oberfläche und/oder zum Benutzer bereitgestellt. Dies schließt Warmmeldungen bei systemkritischen Ausfällen wie beispielsweise ein leerer Vorratsspeicher oder ein undichter Kolben mit ein. Durch Füllstandsensoren (4) an den Vorratsspeichern sowie durch Verwendung von Druckaufnehmern an allen Zuleitungen zur Vermischungseinrichtung können derartige Fehlerquellen detektiert werden.

Des Weiteren werden die Anzahl der Desinfektionsvorgänge dokumentiert und über die Anzeigeeinheit (16) ausgegeben. Die Anzeigeeinheit (16) verfügt zudem über ein Modul, welches erlaubt, alle vom Gerät gespeicherten Daten auf ein anderes Gerät vorzugsweise einen Computer zu transferieren. Die Vorratsspeicher (1-3) sind mit einem Codierungssystem versehen, was die eindeutige Zuordnung der beinhaltenden Flüssigkeit erlaubt. Durch die Steuereinheit (14) wird bei nicht-eindeutig erkannten Flüssigkeiten der Betrieb des Gerätes solange unterbrochen, bis die vorgesehene Flüssigkeit im Vorratsspeicher anliegt.

In Fig. 12 wird eine Ausführungsform der Abgabevorrichtung dargestellt, bei der die Wirklösung als Aerosol auf die zu desinfizierende Oberfläche gebracht wird. Hierbei werden in den Vorratsspeichern (1, 2) aus den Eduktlösungen Aerosole mittels eines Aerosolgenerators (30) erzeugt. Dazu generieren Ventilatoren (19) mittels einer Luftzufuhr (18) oberhalb der Eduktlösungen einen zirkulierenden Luftstrom. Dieser zirkulierende Luftstrom reichert sich mit Flüssigkeitstropfen an, so dass Aerosole oberhalb der Eduktlösungen gebildet werden. Die Eduktaersole werden bei Bedarf über den Luftstrom in eine Vermischungsvorrichtung (10) geleitet, wo die Wirklösung in Form eines Aerosols gebildet wird. Die Wirklösung wird dann in Form eines Aerosols auf die zu desinfizierende Oberfläche gebracht.

In Fig. 13 ist eine Ausführungsform der Abgabevorrichtung dargestellt, bei der die Wirklösung in Form eines Flüssigkeitssprays erst auf der zu desinfizierenden Oberfläche gebildet wird. Hierzu sind die Vorratsspeicher (1, 2) jeweils mit Pumpen (20) ausgestattet. Über die jeweiligen Pumpen (20) werden die Eduktlösungen bei Bedarf durch Düsen (21) geleitet, so dass ein Spray (28) entsteht. In diesem Spray (28) werden die Eduktlösungen vereinigt und gemeinsam auf die zu desinfizierende Oberfläche gebracht.

In Fig. 14 ist eine Vorrichtung dargestellt, bei der die Wirklösung über eine Wasserleitung (22) mittels eines Wasserhahns (23) appliziert wird. Durch Pumpen (24) werden die Edukte in den Wasserfluss (25) eingebracht, wodurch die Wirklösung entsteht. In die Wasserleitung (22) ist ein Durchflusssensor (26) integriert, welcher zur Prozesskontrolle verwendet wird und bei detektiertem Wasserfluss den Zustrom der Edukte (29) aktiviert.

In Fig. 15 ist eine weitere Ausführungsform einer Vorrichtung dargestellt, bei der die Wirklösung über einen Zustrom von Edukten (29) in eine Wasserleitung (25) mittels eines Wasserhahns (23) appliziert wird. Die Edukte werden in dieser Ausführungsform durch eine Venturi-Pumpe (27) in den Wasserfluss eingesogen, im Wasserfluss vermischt

und als Wirklösung über den Wasserhahn (23) auf die zu desinfizierende Oberfläche gegeben.

In Fig. 16 ist eine Ausführungsform einer Vorrichtung dargestellt, bei welcher ein Teil der Edukte in einem Vorratsspeicher (1 oder 2) vorgelegt ist, während ein weiterer Teil der Edukte durch eine in die Abgabevorrichtung integrierte Plasmaquelle (32) erzeugt wird. Durch den Aerosolgenerator (30), den Ventilator (18) und Luftzufuhr wird aus der Eduktlösung ein Aerosol generiert, welches dann in der Abgabevorrichtung durch die Plasmaquelle mit dem weiteren Edukt zur Reaktion gebracht wird. Die so entstandene Wirklösung wird als Aerosol (33) im Applikationsbereich (34) auf die zu desinfizierende Oberfläche gebracht. Des Weiteren verfügt diese Ausführungsform der Vorrichtung über eine Absaugvorrichtung (35), welche mit einem Filter (36) ausgestattet ist, welcher gesundheitsschädliche gasförmige flüssige und gasförmige Bestandteile aus der Raumluft entfernt.

Bezugszeichenliste:

(1) Vorratsspeicher Edukt A
(2) Vorratsspeicher Edukt B
(3) Vorratsspeicher Verdünnungsflüssigkeit C
(4) Füllstands- und Codierungselektronik
(5) Rückschlagventil
(6) Kolben für konzentrierte Lösung Edukt A
(7) Kolben für konzentrierte Lösung Edukt B
(8) Kolben für Verdünnungsflüssigkeit C
(9) Kolben zur Erzeugung von Druckluft
(10) Vermischungsvorrichtung
(11) Abgabevorrichtung in Form einer Düse
(12) Kurbelwelle
(13) Antriebseinheit
(14) Steuerelektronik
(15) Druckkontrollsensor
(16) Anzeigeeinheit für Füllstand und Systemstatus
(17) optischer Sensor für berührungslose Bedienung und Handgrößenerkennung
(18) Zufuhr von Luft.
(19) Ventilator
(20) Pumpe
(21) Düse
(22) Wasserleitung
(23) Wasserhahn
(24) Pumpe zum Wasserfluss
(25) Wasserleitung
(26) Durchflusssensor
(27) Venturi-Pumpe
(28) Spray
(29) Zustrom der Edukte
(30) Aerosolgenerator
(31) Detektor
(32) Plasmaquelle
(33) Aerosol
(34) Applikationsbereich
(35) Absaugvorrichtung
(36) Filter

Experimentelle Untersuchungen

**Bakterizide Wirkung**

Die Wirksamkeit der Wirklösung wurde in Suspensionsversuchen untersucht. Hierzu wurde die Wirklösung erhalten, indem zwei Ausgangsflüssigkeiten A und B miteinander vermischt wurden. Ausgangsflüssigkeit A enthielt hierbei 50 mM $H_2O_2$ und 2% (w/v) oder 10% Citronensäure. Ausgangsflüssigkeit B enthielt 50 mM $NO_2^-$. Um die Wirkung

einschätzen zu können, wurde die Wirksamkeit der Wirklösung mit der von Isopropanol, einem bekannten und weit verbreiteten Desinfektionsmittel, als alleinigem Wirkstoff verglichen. Bei dem Versuch wurden je 0.1 mL Bakterienlösung (E. Coli) mit 1 mL einer Mischung aus dem jeweilig zu testendem Mittel sowie CASO-Boullion (Caseinpepton-Sojamehlpepton-Bouillon, Carl Roth GmbH + Co. KG, Deutschland) vermischt. Die Expositionszeit betrug 30 s. Die CASO-Boullion diente hier zur Verdünnung sowie als künstliche organische Last für den Desinfektionsversuch. In Fig. 5 sind die Ergebnisse dieser Versuche dargestellt. Aus den Resultaten geht klar hervor, dass bei der Verwendung als Desinfektionsmittel in Suspension eine stärkere Ansäuerung die Desinfektionswirkung der Wirklösung verstärkt. Diese erhöhte Wirksamkeit hinsichtlich der Inaktivierung von Mikroorganismen in Folge von der Ansäuerung einer Mischung aus $H_2O_2$ und $NO_2^-$ ist in der Literatur bekannt, siehe (Heaselgrave et al., 2010).

**Händedesinfektionsversuche**

Um die Anwendbarkeit der Wirklösung als Händedesinfektionsmittel zu testen, wurden Händedesinfektionsversuche entsprechend der Norm EN 1500 durchgeführt. Die Testung entsprechend der Norm EN 1500 überprüft, wie ein zu testendes Desinfektionsmittel im Vergleich zum Wirkstoff Isopropanol hinsichtlich seiner Desinfektionswirkung wirkt. Dazu werden die Hände zunächst in eine bakterienhaltige (E. Coli) CASO-Boullion eingetaucht und anschließend entsprechend dem Protokoll mit dem jeweilig zu testenden Mittel desinfiziert.

Dieser Test wurde mit drei unterschiedlichen Wirklösungen durchgeführt, wobei die Ausgangsflüssigkeiten B jeweils identisch waren und die Ausgangsflüssigkeiten A jeweils 50 mM $H_2O_2$ enthielten, jedoch entweder 0.5 %, 2 % oder 10 % Citronensäure.

Bei diesen Testungen wurde nur die Wirklösung mit 2 %iger Citronensäure als wirksamer als Isopropanol eingestuft. Im Gegensatz zu den oben beschriebenen Desinfektionsversuchen in Suspension, führt einer stärkere Ansäuerung der Ausgangslösung nicht zu einer Verbesserung, sondern zu einer Verschlechterung der Wirkung.

Weiterhin wurden bei diesen Untersuchungen pH-Messungen durchgeführt. In Fig. 5 sind drei Typen von pH-Messungen dargestellt. Dies sind der pH-Wert der Probanden vor der Behandlung auf der Innenseite der Handfläche (Vorwert), die pH-Werte in den zusammengemischten Wirklösungen und die pH-Werte auf der Haut nach Durchführung der Handdesinfektion entsprechend EN 1500. Bei den beiden letzteren wurde jeweils mit 0.5%, 2% und 10% Citronensäurekonzentration in Wirklösung B getestet.

**Abhängigkeit der Wirksamkeit von Dosis und Expositionszeit**

Um eine Regel aufstellen zu können, welche es erlaubt, die Wirksamkeit einer Wirklösung aus $H_2O_2$ und $NO_2^-$ vorhersagen zu können, muss die Abhängigkeit der Wirksamkeit von Dosis und Expositionszeit bekannt sein. In vielen Fällen kann der Wirksamkeitsparameter *W* entsprechend *der Haberschen Regel* als Produkt aus Dosis und Einwirkzeit $Z_E$ beschrieben werden. Im vorliegenden Fall entstehen eine Vielzahl reaktiver Spezies mit unterschiedlichen Lebenszeiten, sodass diese Abhängigkeit nicht eindeutig ist.

Es seien *P* die für die Desinfektionswirkung relevanten Reaktionsprodukte mit Konzentration [P] des in Reaktion (1) beschrieben Prozesses. Die Habersche Regel ergibt nun den Haberschen Wirksamkeitsparameter

$$\widehat{W} = \int_{t_1}^{t_2} [P] dt. \qquad (23)$$

Für die kurzlebigen, für die Desinfektionswirkung relevanten Reaktionsprodukte P kann angenommen werden, dass diese eine Lebenszeit $\tau$ besitzen und nach einer Zeit t mit der Wahrscheinlichkeit g(t)

$$g(t) = \exp\left(-\frac{t}{\tau}\right) \qquad (24)$$

noch nicht zerfallen sind. Somit ist

$$Q(t) = c_1 \times k_1 [NO_2^-][H_2O_2] \qquad (25)$$

der Quellterm der für die Desinfektionswirkung relevanten Reaktionsprodukte, wobei $c_1$ der auf die Reaktionsrate bezogene Anteil der für die Desinfektionswirkung relevanten Reaktionsprodukte ist.

Die Konzentration [P] lässt sich nun durch die Faltung

$$[P] = Q * g = \int Q(\tau)g(t-\tau)d\tau \quad (26)$$

angeben. Unter der Annahme, dass die Lebenszeit τ der für die Desinfektionswirkung relevanten Reaktionsprodukte, welche in Folge von Reaktion (1) gebildet werden, viel kürzer ist, als die Änderung der Konzentrationen von $NO_2^-$ und $H_2O_2$, zum Beispiel $\left|\frac{\tau}{[H_2O_2](t=0)}\frac{d[H_2O_2](t)}{dt}\right| \ll 1$, kann *g*(*t*) näherungsweise durch eine Dirac-Distribution in der Form *g(t)* = $c_2 \times \delta(t)$ beschrieben werden, sodass für den Wirksamkeitsparameter W gilt:

$$\widehat{W} = c_1 \times c_2 \int_{t_1}^{t_2} k_1 \times [H_2O_2] \times [NO_2^-]\, dt \quad (27)$$

Der Vorfaktor $c_1 \times c_2$ in Gleichung (26) bewirkt lediglich eine Skalierung. Zur Vereinfachung wird daher der *Wirksamkeitsparameter*

$$W = \int_{t_1}^{t_2} k_1 \times [H_2O_2] \times [NO_2^-]\, dt \quad (28)$$

definiert, mit welchem eine notwendige und hinreichende Bedingung für die Wirksamkeit des erfindungsgemäß hergestellten Desinfektionsmittels angegeben werden kann. Die Wirksamkeit einer Mischung aus $NO_2^-$ und $H_2O_2$ in Abhängigkeit des so definierten Wirksamkeitsparameters W wurde im Rahmen der dieser Erfindung zugrundeliegenden Untersuchungen bestimmt.

Die Gültigkeit von Gleichung (5) wird durch folgendes Experiment bestätigt:
Zu einer Bakterienlösung mit einem Volumen von 0.1 mL werden 0.22 mL CASO-Boullion hinzugegeben. Diese Bakterienlösung wird für einen Einwirkzeitraum $Z_E$ einer Wirklösung aus $H_2O_2$, $NO_2^-$ und pH-Puffer ausgesetzt, wobei das Volumen der Wirklösung 0.78 mL beträgt. In Fig. 7 ist der errechnete Wirksamkeitsparameter zusammen mit den experimentellen Resultaten dargestellt, welche die Gültigkeit der Beziehung (5) bestätigen.

Mit den Formeln (5) bis (15) ergeben sich die Konzentrationen von $H_2O_2$ und $NO_2^-$ Da es sich bei diesen Versuchen um Suspensionsversuche handelt, wurde die Ausgasung vernachlässigt, also in (11) und (12) r=0 angenommen.

Aus den im Rahmen dieser Erfindung gewonnenen Daten ergibt sich, dass für den Wirksamkeitsparameter (5) in Suspension zur Inaktivierung von E. coli die Bedingung *W* > 0,3 *mM,* idealerweise *W* > 0,5 *mM,*
gelten muss, damit eine Desinfektionswirkung erzielt werden kann. Auf die notwendigen Wirksamkeitsparameter für Bakteriensporen und unbehüllte Viren wird weiter unten eingegangen.

**Verteilungsschritt und Wirksamkeitskurven**

Bei dem Einsatz der Wirklösung zur Oberflächendesinfektion muss stets ein Verteilungsschritt durchgeführt werden, bei welchem die Wirklösung auf der zu desinfizierenden Oberfläche aufgebracht und/oder verteilt wird. Beispielsweise wird die Wirklösung beim Einsatz als Handdesinfektionsmittel typischerweise für eine gewisse Zeit, üblicherweise 30 s, verrieben. Zudem dauert es bei unebenen oder porösen Oberflächen eine gewisse Zeit, bis die Wirklösung durch Diffusion ausreichend in die Oberfläche eingedrungen ist. Eine effektive Oberflächendesinfektion ist nur gewährleistet, wenn die Wirkflüssigkeit nach Beendigung des Verteilungsschrittes, also dann, wenn die zu desinfizierende Oberfläche nach dem Verarbeitungszeitraum $Z_A$ restlos benetzt ist, noch für eine Einwirkzeit $Z_E$ auf der Oberfläche verbleibt und während dieser Einwirkzeit $Z_E$ noch ausreichend wirksam ist.

Dies ist nach Gleichung (5) für E. coli genau dann erfüllt, wenn

$$W = \int_{t_1}^{t_2} k_1 \times [H_2O_2] \times [NO_2^-]\, dt \geq 0{,}3 \;\; \text{besser} \geq 0{,}5\ \text{mM.} \quad (29)$$

Der Wirksamkeitsparameter, berechnet nach den Formeln (5) bis (16), ist für $Z_A$=30 s und $Z_E$=15 s in Fig. 7 durch Isolinien dargestellt. Um die Gültigkeit dieses Zusammenhanges zu überprüfen, wurde das folgende Experiment durchgeführt:
Jeweils 0.39 mL zweier Ausgangsflüssigkeiten A und B wurden mit 0.22 mL CASO-Boullion gemischt. Hierbei enthielt die Ausgangsflüssigkeit A 50 mM $H_2O_2$ sowie eine Pufferlösung und Ausgangsflüssigkeit B enthielt 50 mM $NO_2^-$ Die Pufferlösung dient hierbei zum Einstellen des pH-Wertes der Mischung aus Wirklösung und CASO-Boullion. Nach 30

s wurden zu dieser Mischung 0.1 mL einer Bakteriensuspension (E. coli) hinzugegeben. Nach einer Expositionszeit von 15 s wurde die erhaltene Suspension ausplattiert. In Fig. 7 ist die so erhaltene $log_{10}$-Bakterienreduktion dargestellt. Wie zu erkennen ist, ist höchstens eine Reduktion koloniebildender Einheiten um bis zu 2 $log_{10}$-Stufen zu erreichen, falls 0,15 mM <W < 0,3 mM ist. Wenn 0,3 mM <W < 0,5 mM ist, sind 2 bis 5 $log_{10}$-Stufen zu erreichen. Wenn W>0,5 mM, werden bis zu 7 $log_{10}$-Stufen erreicht. Die gute Übereinstimmung mit den experimentellen Daten mit dem nach (13) definierten Wirksamkeitsparameter unterstreicht die Validität der durch die Formeln (23) - (28) beschrieben Dosis-Wirk-Beziehung für die Nutzung von $NO_2^-$ und $H_2O_2$ als Desinfektionsmittel. Während der Einsatz des Verfahrens mit einem Wirksamkeitsparameter 0,15 mM <W < 0,3 mM insbesondere als unterstützende Hygienemaßnahme, beispielsweise durch Vorschaltung einer entsprechenden Vorrichtung vor einem Wasserhahn sinnvoll ist, kann das Verfahren für W>0,3 mM, besser W>0,5 mM zur hygienischen Oberflächen- und insbesondere zur Handdesinfektion genutzt werden. Für eine sporizide und viruzide Wirkung sind höhere Werte von W notwendig (siehe unten).

In Fig. 8 sind zudem die Start- und End-pH-Werte durch Pfeile dargestellt. Diese wurden durch pH-Messung in den oben beschriebenen Handdesinfektionsversuchen ermittelt. Die Anfangskonzentration der Edukte in der Wirklösung betrug $[H_2O_2]_0$ = $[NO_2^-]_0$=25 mM. Hierbei ist der Startwert jeweils der pH-Wert in der Wirklösung vor Beginn der Handdesinfektion und der Endwert der pH-Wert der Wirklösung auf der Haut nach der Handdesinfektion entsprechend der Norm EN 1500. Um eine Oberflächendesinfektion in Situationen sicherstellen zu können, in welchen - wie bei der Handdesinfektion - eine pH-puffernde Oberfläche vorliegt, müsste idealerweise der zeitliche Verlauf des pH-Wertes bekannt sein, sodass Gleichung (27) integriert werden kann. In praktischen Anwendungen ist die Bestimmung dieser Zeitabhängigkeit jedoch oft nicht möglich. Um dennoch einen sicheren Prozess gewährleisten zu können, genügt es jedoch, wenn - wie hier - der Start und End-pH-Wert angegeben werden kann. Unter der Annahme, dass der pH-Wert während des Prozesses monoton steigt, kann eine ausreichende Desinfektionswirkung in jedem Fall gewährleistet werden, wenn das Integral (5) sowohl mit dem Start- als auch dem End-pH-Wert einen ausreichend großen Wirksamkeitsparameter ergibt. Dies ist, wie in Fig. 8 zu sehen, nur für die Wirklösung der Fall, bei welcher die Ausgangsflüssigkeit 2 % Citronensäure beinhaltet ($W \geq 0.5$). Genau diese Wirklösung bestand als einzige der drei getesteten Lösungen die Norm EN 1500.

Eine alternative wäre hier, die Ausgangslösung A so stark zu puffern, dass der pH-Wert während des Prozesses nahezu konstant gehalten werden kann. Dies ist jedoch insbesondere bei der Verwendung zur Handdesinfektion nachteilhaft, wie unten erläutert.

Bei diesen Experimenten wurden die Anfangskonzentrationen $[H_2O_2]_0$ = $[NO_2^-]_0$ gewählt. Dies ist vorteilhaft, da die Ausgangsstoffe $H_2O_2$ und $NO_2^-$ nahezu vollständig abgebaut werden. In der Flüssigkeit verbleibt lediglich $NO_3^-$ in gesundheitlich und ökotoxisch unproblematischen Konzentrationen. Bei der Verwendung als Oberflächendesinfektionsmittel kann $[H_2O_2]_0$ vorteilhafterweise bis zu 10% niedriger als $[NO_2^-]_0$ gewählt werden, damit möglichst wenig $H_2O_2$ aufgrund der Ausgasung von $NO_2^-$ in der abgeklungenen Wirklösung übrigbleibt.

Es ist auch möglich, $H_2O_2$ wesentlich höher zu konzentrieren, um einen ausreichend großen Wirksamkeitsparameter bei höheren pH-Werten zu erhalten. Dies kann insbesondere bei der Anwendung auf pH-empfindlichen Oberflächen sinnvoll sein.

**Ausgasung**

In Fig. 9 (links) ist der Messaufbau zur Quantifizierung der Gesamtmenge von ausgegastem $NO_2$ dargestellt. Eine Petrischale wurde mit 10 mL der Desinfektionsflüssigkeit befüllt und in einen Behälter gestellt, welcher mit einem Gasfluss von 1 slm durchströmt wird. Durch Integration der so ermittelten $NO_2$-Erzeugungsrate (Fig. 9 rechts), wurde ermittelt, dass in diesem Fall bis zu 3 % des sich in der Flüssigkeit befindenden $NO_2^-$ als $NO_2$ ausgast.

Die Ausgasung wirkt sich auch auf den Ablauf der chemischen Reaktionen in der Flüssigkeit aus, wie das folgende Experiment zeigt. Wie in Fig. 10 dargestellt, wurden $H_2O_2$ und $NO_2^-$ mit Citronensäure mit einer Konzentration von jeweils 50 mM und einem Volumen von jeweils 3 mL in einem Becherglas bei einem pH-Wert von 2,8 für eine Sekunde vermischt. Direkt danach wurden 2 mL der Mischung aus dem Becherglas in ein flaches Gefäß mit 15 cm Durchmesser gegeben, um eine Verteilung der Flüssigkeit ähnlich einer Oberflächendesinfektion zu erreichen.

Nach einer Reaktionszeit von 180 s wurden in beiden Flüssigkeiten mittels Teststreifen die Konzentrationen von $H_2O_2$ und $NO_2^-$ bestimmt. Während im Becherglas die Konzentrationen von $NO_2^-$ und $H_2O_2$ kleiner als 300 $\mu$M sind, ist beträgt die Konzentration von $H_2O_2$ im flachen Gefäß 2,5 mM, während kein $NO_2^-$ mehr nachweisbar ist. Durch den Konzentrationsunterschied lässt sich der zuvor freie Parameter r in den Ausdrücken (19) und (20) für diese Oberflächenanwendung als r = 0,11 bestimmen, was bedeutet, dass 9.9 % (ergibt sich aus 0,11/(1+0,11)) des eingebrachten $NO_2^-$ als $NO_2$ ausgasten.

Aufgrund der genannten Daten zur Ausgasung wird angenommen, dass typischerweise bis zu 10% der verwendeten Menge von $NO_2^-$ als $NO_2$ ausgasen kann.

Bei einer typischen Applikationsmenge von 3 mL der Wirksubstanz und einer Anfangskonzentration von $NO_2^-$ von 300 mM in der gemischten Wirklösung würden demnach bis zu 118 $\mu$mol $NO_2^-$ pro Applikation freigesetzt. In einem

Raum ohne Lüftung mit einem Volumen von $10m^3$ wäre der MAK-Wert nach zweimaliger Anwendung überschritten.

**Mutagenität**

Es wurde ein Ames-Test zur Untersuchung der Mutagenität des Desinfektionsproduktes durchgeführt. Der Test basiert darauf, dass einem E. coli-Stamm durch eine Punktmutation die Fähigkeit genommen wird, auf einem gegebenen Nährboden zu wachsen. Durch Hinzugabe eines Mutagens bilden sich Revertanten, welche zum Wachstum auf dem Nährmedium in der Lage sind. Auch ohne Zugabe eines Mutagens entstehen durch spontane Mutationen einige wenige Revertanten (natürliche Mutagenität).

Der Mutagenitätsversuch wurde mit zwei Lösungen aus 50 mM $NO_2^-$ sowie 50 mM $H_2O_2$, angesäuert mit 2% Citronensäure, durchgeführt. Diese ersten Testungen ergaben keine Mutagenität (Mutagenitätslevel entspricht etwa der natürlichen Mutagenität) für das Produkt.

**pH- und Temperatur-Abhängigkeit**

Ändert sich der pH-Wert und/oder die Temperatur während des Prozesses, etwa durch das Aufbringen der Flüssigkeit auf eine puffernde und/oder geheizte Oberfläche, ist dies bei der Auswertung der Dichten (Gleichungen (11) und (12)) und entsprechend des Integrals (5) mit einem zeitabhängigen pH Wert pH(t) bzw. einer zeitabhängigen Temperatur T(t) zu berücksichtigen. Insbesondere kann dazu das Integral (5) bei aufeinanderfolgenden Prozessschritten stückweise für entsprechende Temperaturen und pH-Werte berechnet werden. Für die Messung des pH-Wert-Verlaufes in Suspension eignet sich hierbei insbesondere UV-Spektroskopie. Hierzu kann der pH-Wert aus dem Verhältnis von $NO_2^-$ zu $HNO_2$ in der Flüssigkeit bestimmt werden. Ebenfalls anwendbar sind hierzu übliche pH-Sonden. Die pH-Messung der auf eine Oberfläche aufgebrachten Flüssigkeit kann ebenfalls durch die Verwendung entsprechender pH-Sonden realisiert werden. Für die pH-Oberflächenmessungen wurden bei den in dieser Schrift vorgestellten Handdesinfektionsversuchen eine pH-Oberflächenelektrode der Firma Mettler-Toledo International Inc. verwendet (pH Elektrode InLab Surface). Zur Temperaturmessung stehen unterschiedlichste Methoden zur Verfügung. Die in dieser Arbeit angegebenen Temperaturwerte wurden mittels eines fiberoptischen Temperatursensors mit schneller Ansprechzeit und geringer Wärmekapazität ermittelt (FOTEMP1-OEM der Firma Weidmann Technologies Deutschland GmbH).

Die Temperaturabhängigkeit des Reaktionskoeffizienten $k_1$ wurde mittels UV-Absorptionsspektroskopie bestimmt. Dazu wurden bei Temperaturen zwischen 0°C und 40°C die Reaktanden $NaNO_2$ und $H_2O_2$ unter Verwendung eines Citronensäure-Phosphat-Puffers zur Reaktion gebracht. Die Reaktionsrate wurde durch zeitaufgelöste Quantifizierung der Dichten von $NO_2^-$ und $HNO_2$ ermittelt. Zur Ermittlung der Reaktionsraten wurden Ausgangskonzentrationen von $H_2O_2$ und $NO_2^-$ von jeweils 5 mM verwendet. In Abbildung 17 ist der entsprechende Arrhenius-Graph dargestellt. Es ergibt sich der temperaturabhängige Reaktionskoeffizient

$$k_4 = 3{,}56 \cdot 10^{14} \exp\left(-\frac{E_A}{RT}\right) M^{-1} s^{-1}$$

mit der effektiven Aktivierungsenergie $E_A$=70 kJ/mol.

**Keine Bildung** von **Peroxinitrat**

Die nachfolgend dargestellten Versuche zeigen, dass bei der Durchführung des erfindungsgemäßen Desinfektionsverfahrens kein Peroxinitrat entsteht.

Im Eisbad wurden in Reaktionslösungen aus 73,2 mM $NaNO_2$, 58,9 mM $H_2O_2$ und 100 mM HCl auf 0°C abgekühlt. Je 1 mL der Reaktionslösungen wurden in einem Reaktionsgefäß (ebenfalls im Eisbad) vermischt. Die Salzsäure dient hierbei zur Absenkung des anfänglichen pH-Wertes der Mischung auf den Wert 2,1. Nach 5 Minuten im Eisbad wurden die Reaktionsprodukte mittels UV-Spektroskopie quantifiziert. Die gemessene Absorbanz Aexp = -ln(I/I0) ist in Abbildung 18 dargestellt. Hierbei stellen I0 und I die gemessenen Intensitäten vor bzw. nach Durchgang durch das Testmedium dar. Zur Quantifizierung der Reaktionsprodukte wurden die Konzentrationen [i] wellenlängenabhängigen Wirkungsquerschnitte $\sigma_i(\lambda)$ der Spezies $i \in \{NO_2^-, HNO_2, NO_3^-, H_2O_2\}$ im Wellenlängenbereich 250 nm ≤ λ ≤ 420 nm mittels der Methode der kleinsten Quadrate mittels der Modellfunktion ("fit") $A = \Sigma_i[i]\sigma_i L$ variiert, sodass sich die beste Übereinstimmung zwischen Modellfunktion A und experimentell ermittelter Absorbanz $A_{exp}$ ergibt. In der Mischung wurden nach 5 Minuten Reaktionszeit auf diese Art und Weise 19 mM $NO_2$ und 4,8 mM $HNO_2$ ermittelt. $H_2O_2$ und $NO_2^-$ wurden nicht detektiert. Ebenfalls kann ausgeschlossen werden, dass Peroxinitrat gebildet wurde, welches im Bereich

230-280 nm absorbiert (Loegager & Sehested 2005). Die Ergebnisse sind stöchiometrisch konsistent mit denen durch Reaktionen (2) bis (4) beschrieben Reaktionsmechanismen.

In einem weiteren Versuch wurden die gleichen Ausgangslösungen wie oben beschrieben ($NaNO_2$, $H_2O_2$, HCl) genutzt, welche ebenfalls im Eisbad auf eine Temperatur von 0° C gebracht wurden. Diese wurden bei diesem Versuch jedoch direkt in einer Küvette zur Reaktion gebracht, sodass die Reaktion zeitaufgelöst verfolgt werden konnte. Hierzu wurden die $NaNO_2$ und HCl-Lösungen in der Küvette vorgelegt und die Reaktion durch Zugabe von HCl gestartet. Die Ergebnisse dieser Messung sind in Abbildung 19 dargestellt. Zudem ist in Abbildung 20 die Messung sowie die angepasste Modellfunktion mit Beiträgen der Spezies $NO_3^-$, ONOOH sowie $HNO_2/NO_2^-$ dargestellt. $HNO_2/NO_2^-$ bezeichnet hierbei die Gesamtdichte von $HNO_2$ und $NO_2^-$. Der Wirkungsquerschnitt der Säure bzw. der konjugierten Base wurde hierzu im Vorfeld für den pH-Wert 2,1 berechnet. Der verwendete Wirkungsquerschnitt für ONOOH wurde in eigenen Vorarbeiten ermittelt und ist konsistent mit dem in Loegager & Sehested (1993) veröffentlichen Wirkungsquerschnitt. Die Bildung von ONOOH wurde somit auch im Grenzbereich des erfindungsgemäß durchgeführten Verfahrens (0°C, pH 2,1) nachgewiesen.

### Wirksamkeitsparameter für verschiedene Mikroorganismen

Die biologische Struktur verschiedener Arten von Mikroorganismen ist unterschiedlich. Dies legt nahe, dass nicht alle Mikroorganismen gleich auf ein Desinfektionsverfahren reagieren. Um die Gültigkeit des Wirksamkeitsparameters für verschiedene Mikroorganismen-Kategorien zu prüfen, wurde die Wirkung des erfindungsgemäßen Verfahrens an vegetativen und sporenbildenden Bakterien sowie an unbehüllten Viren getestet. Im Gegensatz zu vegetativen Bakterien sind Bakteriensporen und unbehüllte Viren mit alkoholbasierten Mitteln nicht oder nur nach unzureichend langer Zeit inaktivierbar. Diese Widerstandsfähigkeit zeigt sich auch in den in Abb. 21 gezeigten Versuchsergebnissen. Bereits bei einem Wirksamkeitsparameter von 0,3 mM werden 99% (2 log-Stufen) von *Escherichia coli* Bakterien in Suspension abgetötet. Weitere Spezies der Kategorie "vegetative Bakterien" wie *Pseudomonas aeruginosa* und *Staphylokokkus aureus* benötigen zwar einen etwas höheren Wirksamkeitsparameter von 1,5 mM bzw. 1,7 mM, jedoch liegen diese Werte noch deutlich unter den benötigten Wirksamkeitsparametern für Bakteriensporen. Hier ist für *Clostridium difficile* Sporen ein Wirksamkeitsparameter von 10 mM für eine 99%ige Inaktivierung notwendig. Der Keim *Clostridium difficile* besitzt eine besondere Relevanz, da dieser der häufigste Erreger nosokomialer und Antibiotika-assoziierter Durchfallerkrankungen ist (Lübbert et al. 2014). Widerstandsfähigere Sporen wie *Bacillus atrophaeus* benötigen einen Wirksamkeitsparameter von 30 mM. Für die dritte untersuchte Kategorie "unbehüllte Viren" wurde die Desinfektionsleistung am Poliovirus getestet, der für eine 99%ige Inaktivierung einen Wirksamkeitsparameter von 17,2 mM erfordert. Die jeweiligen Werte sind zur Übersicht in der nachfolgenden Tabelle 1 aufgeführt.

Tabelle 1: Wirksamkeitsparameter für vegetative Mikroorganismen, Bakteriensporen und unbehüllte Viren

| Art | Mikroorganismus | notwendiger Wirksamkeits-parameter für 2-log (99%) Inaktivierung /mM | notwendiger Wirksamkeits-parameter für 4-log (99,99%) Inaktivierung /mM |
|---|---|---|---|
| vegetative Mikroorganismen | E. coli | 0,3 | 0,42 |
| | P. aeruginosa | 1,5 | 1,62 |
| | S. aureus | 1,7 | 3,2 |
| Bakteriensporen | C. difficile | 10 | 12,7 |
| | B. atrophaeus | 30 | 52 |
| unbehüllte Viren | Poliovirus Typ 1 | 17,2 | 24,2 |

### Wirksamkeitsparameter für verschiedene Mikroorganismen

In Heaselgrave 2010 sind mikrobiologische Inaktivierungsversuche für verschiedene Mikroorganismen bei

folgenden Parametern durchgeführt worden: pH = 5, $[H_2O_2]_0$ = 171 mM und $[NO_2^-]_0$ = 29 mM. Die nachfolgende Tabelle 2 zeigt die aus diesen Parametern und der entsprechenden Einwirkzeit berechneten Wirksamkeitsparameter W.

Tabelle 2: Wirksamkeitsparameter W für das Desinfektionsmittel nach Heaselgrave 2010 für eine Einwirkzeit von 15 min bzw. 60 min sowie für eine erfindungsgemäße Verarbeitungs- und Einwirkzeit ($Z_A$ = 2 s, $Z_E$ = 90s).

| | Heaselgrave 2010 | | | |
|---|---|---|---|---|
| **Mikroorganismus** | **benötigte Einwirkzeit für 4log Reduktion /s** | **berechneter Wirksamkeits-parameter für 4log Reduktion / mM** | **berechneter Wirksamkeitsparameter bei $Z_A$ = 2 s, $Z_E$ = 90 s / mM** | **Effizienz bei $Z_A$ = 2 s, $Z_E$ = 90 s / %** |
| P. aeruginosa | 900 | 4,6 | 0,51 | 1,9 |
| S. aureus | 900 | 4,6 | 0,51 | 1,9 |
| B. subtilis Sporen | 3600 | 14,2 | 0,51 | 1,9 |

Für vegetative Mikroorganismen wie *Pseudomonas aeruginosa* oder *Staphylokokkus aureus* werden von Heaselgrave et al. Wirksamkeitsparameter von 4,6 mM benötigt, um einen Reduktionsfaktor von 4 log-Stufen zu erreichen. Für Bakteriensporen, die wesentlich widerstandsfähiger sind, liegt der benötigte Wirksamkeitsparameter deutlich höher. Gemäß den Versuchsparametern von Heaselgrave et al. (2010) berechnet sich der Wirksamkeitsparameter für *Bacillus subtilis* Endosporen entsprechend zu 14,2 mM. Im Gegensatz zum erfindungsgemäßen Desinfektionsverfahren werden zum Erreichen der Wirksamkeitsparameter von 4,6 mM bzw. 14,2 mM viel längere Zeiträume (15 min bzw. 60 min) benötigt.

In der Tabelle sind zusätzlich die Wirksamkeitsparameter angegeben, die sich rechnerisch aus den von Heaselgrave et al. (2010) verwendeten Parametern (pH, $C_{H2O2}$, $C_{NaNO2}$) bei einer Verarbeitungszeit $Z_A$ = 2 s und einer Einwirkzeit $Z_E$ = 90 s ergeben würden. Da die Parameter bei allen Versuchen gleich waren, ergibt sich für alle dargestellten Mikroorgansimen ein identischer Wirksamkeitsparameter von 0.51 mM. Dieser ist für vegetative Mikroorganismen 9-mal und für Endosporen 28-mal kleiner als es für eine Reduktion um 4 log-Stufen notwendig wäre. Eine Inaktivierung von relevanten Mikroorganismen innerhalb von 92 s ist mit den von Heaselgrave et al. gewählten Parametern völlig aussichtslos, obwohl die von Heaselgrave et al. verwendeten $NaNO_2$ und $H_2O_2$-Konzentrationen im Vergleich zu den erfindungsgemäßen Konzentrationen bereits sehr hoch angesetzt sind. Alle anderen erfindungsgemäßen Kombinationen aus Verarbeitungs- und Einwirkzeit mit den initialen Konzentrationen nach Heaselgrave et al. liefern noch geringere Wirksamkeitsparameter und erhöhen damit den notwendigen Faktor zur Erreichung eines ausreichenden Wirksamkeitsparameters.

Des Weiteren ist die Effizienz des Prozesses von Heaselgrave et al. für den betrachteten Zeitraum von 92 s mit E = 1,9 % äußerst gering. Das bedeutet, dass bei der betrachteten Verarbeitungs- und Einwirkzeit ($Z_A$ = 2 s, $Z_E$ = 90 s) der von Heaselgrave et al. erreichte Wirksamkeitsparameter nur 1,9% des Wirksamkeitsparameters entspricht, der theoretisch bei einer günstigen Wahl von pH-Wert und Ausgangskonzentrationen von $H_2O_2$ und $NO_2^-$ erreichbar wäre. Neben der besseren Wirksamkeit ist vor dem Hintergrund einer effizienten Rohstoffnutzung daher auch im Punkt Effizienz das erfindungsgemäße Verfahren dem von Heaselgrave et al. vorzuziehen.

Literaturliste:

Anbar, M., & Taube, H. (1954). Interaction of Nitrous Acid with Hydrogen Peroxide and with Water. Journal of the American Chemical Society, 76(24), 6243-6247. https://doi. org/10.1021 /ja01653a007

Damschen, D. E., & Martin, L. R. (1983). Aqueous aerosol oxidation of nitrous acid by O2, O3AND H2O2. Atmospheric Environment (1967), 17(10), 2005-2011. https://doi.org/10.1016/0004-6981(83)90357-8

E. Halfpenny, P. L. R. (1952). Pernitrous acid. The reaction between hydrogen peroxide and nitrous acid, and the properties of an intermediate product. Journal of the Chemical Society (Resumed), (0), 928-938.

Heaselgrave, W., Andrew, P. W., & Kilvington, S. (2010). Acidified nitrite enhances hydrogen peroxide disinfection of Acanthamoeba, bacteria and fungi. Journal of Antimicrobial Chemotherapy, 65(6), 1207-1214. https://doi.org/10.1093/jac/dkq075

Ikawa, S., Tani, A., Nakashima Y., Kitano K. (2016) Physicochemical properties of bactericidal plasma-treated water. Journal of Physics D: Applied Physics, 49, 425401.

Jiang, G., & Yuan, Z. (2013). Synergistic inactivation of anaerobic wastewater biofilm by free nitrous acid and hydrogen peroxide. Journal of Hazardous Materials, 250-251, 91-98. https://doi.org/10.1016/j.jhazmat.2013.01.047

Lammel G., Perner, D., Warneck, P. (1990) Decomposition of pernitric acid in aqueous solution. Journal of Physical Chemistry, 94, 6141.

Lee, Y.-N., & Lind, J. A. (1986). Kinetics of aqueous-phase oxidation of nitrogen(III) by hydrogen peroxide. Journal of Geophysical Research, 91(D2), 2793. https://doi.org/10.1029/JD091iD02p02793

Loegager, T., & Sehested, K. (2005). Formation and decay of peroxynitric acid: a pulse radiolysis study. The Journal of Physical Chemistry, 97(39), 10047-10052

Loegager, T., & Sehested, K. (1993). Formation and decay of peroxynitrous acid: a pulse radiolysis study. The Journal of Physical Chemistry, 97(25), 6664-6669

Lübbert C, John E, von Müller L (2014) Clostridium difficile infection-guideline-based diagnosis and treatment. Deutsches Ärzteblatt International, 111, 723.

Lukes, P., Dolezalova, E., Clupek, M., Jablonowski, H., Woedtke, T. Von, & Reuter, S. (2015). Kinetics of peroxynitrite formation and its decomposition in air plasma treated liquids, 5-8.

Szabo, J. G., Adcock, N. J., & Rice, E. W. (2014). Disinfection of Bacillus spores with acidified nitrite. Chemosphere, 113, 171-174. https://doi.org/10.1016/j.chemosphere.2014.05.038

Vione, D., Maurino, V., Minero, C., Borghesi, D., Lucchiari, M., & Pelizzetti, E. (2003). New processes in the environmental chemistry of nitrite. 2. The role of hydrogen peroxide. Environmental Science and Technology, 37(20), 4635-4641. https://doi. org/10.1021 /es0300259

EP 1 715 057 " Planar device and method for generating a plasma or reactive species"

US 20170172149 "Sterilization method, formulation for sterilization use, and device for producing sterilizing liquid"

WO 2011134010 "Control of bacterial activity, such as in sewers and wastewater treatment systems"

## Patentansprüche

**1.** Ein Desinfektionsverfahren aufweisend wenigstens die Edukte $H_2O_2$ und $NO_2^-$ bestehend aus mehreren Teilschritten umfassend wenigstens:

- einen Vermischungsschritt, wobei die Edukte zum Erhalt einer Wirklösung vermischt werden;
- einen Verteilungsschritt, bei dem die Wirklösung auf einer zu desinfizierenden Oberfläche verteilt wird, wobei der Vermischungsschritt und der Verteilungsschritt in einem Verarbeitungszeitraum $Z_A$ stattfinden, der zum Zeitpunkt to beginnt, wenn die Edukte zuerst miteinander in Kontakt gebracht werden, und zum Zeitpunkt $t_1$, bei dem jeder Punkt auf der zu desinfizierenden Oberfläche mit Wirklösung benetzt ist, endet, wobei $t_0$ gleich 0 ist und $t_1$ größer als to ist,

und

- darauffolgend einen Einwirkschritt, bei dem die verteilte Wirklösung über einen Einwirkzeitraum $Z_e$, der zum Zeitpunkt $t_1$ beginnt und nach der Zeitspanne $Z_e$ zum Zeitpunkt $t_2$ endet, auf der mit Wirklösung kontaktierten Oberfläche einwirkt,

wobei $t_2$ den Zeitpunkt darstellt, an dem jeder Punkt auf der mit Wirklösung kontaktierten Oberfläche ausreichend lange mit Wirklösung benetzt ist, um eine desinfizierende Wirkung zu erhalten, und wobei und $t_2$ größer als $t_1$ ist, **gekennzeichnet dadurch, dass**

die maximale $NO_2^-$ Konzentration $[NO_2^-]_0$ zum Zeitpunkt $t_0$ des Vermischungsschritts bei 300 mM liegt, die zeitabhängigen Konzentrationen der Edukte $[H_2O_2]$ und $[NO_2^-]$ während des Verarbeitungszeitraumes und des Einwirkzeitraumes durch

$$[NO_2^-] = \frac{A}{k_1} \text{ und } [H_2O_2] = \frac{A+D}{k_1 + rk_1}$$

gegeben sind, wobei

$$A = -\frac{D}{1-\exp(D(t-C))}, \quad C = -\frac{\ln\left(\frac{D}{[NO_2^-]_0 \cdot k_1}+1\right)}{D}, \quad D = [H_2O_2]_0 \cdot (k_1 + rk_1) - [NO_2^-]_0 \cdot k_1,$$

$$k_1 = k_4 \frac{[H_3O^+]^2}{\left(K_{S,H_3O_2^+} + [H_3O^+]\right)\left(K_{S,HNO_2} + [H_3O^+]\right)}, \quad k_4 = 3.56 \cdot 10^{14} \exp\left(-\frac{E_A}{RT}\right) M^{-1}s^{-1},$$

$$K_{S,HNO_2} = 5{,}13 \times 10^{-4},\ K_{S,H_3O_2^+} = 2 \times 10^{-2},\ [H_3O^+] = 10^{-pH},\ E_A = 70 \text{ kJ/mol}$$

bezeichnet und $[H_2O_2]_0$ die initiale $H_2O_2$ Konzentration zum Zeitpunkt $t_0$ des Vermischungsschrittes bezeichnet und
wobei $r$ = 0,11 zu setzten ist, und
die zeitintegrierte Reaktionsrate W über den Einwirkzeitraum $Z_e$ die Ungleichung

$$W = \int_{t_1}^{t_2} k_1 \cdot [H_2O_2] \cdot [NO_2^-]\, dt \geq 10\ mM$$

erfüllt,

wobei $t_2$ 3 Minuten nicht übersteigt, und
wobei $k_1$ die Geschwindigkeitskonstante der Reaktion zwischen $H_2O_2$ und $NO_2^-$ bezeichnet,
wobei $k_1$ vom pH-Wert der Wirklösung auf der Oberfläche abhängt und
wobei der pH-Wert der Wirklösung auf der Oberfläche und die Temperatur eine Zeitabhängigkeit aufweisen können, und
wobei der pH-Wert der Wirklösung vor Kontakt mit der zu desinfizierenden Oberfläche und der pH-Wert der Wirklösung auf der mit Wirklösung kontaktierten Oberfläche im Bereich von $2{,}1 \leq pH < 6{,}8$ liegen.

**2.** Das Desinfektionsverfahren nach Anspruch 1, wobei der Verarbeitungszeitraum, der zum Zeitpunkt $t_1$ endet, ausgewählt ist aus dem Bereich $0 < t_1 \leq 75$ s, insbesondere ausgewählt ist aus dem Bereich $0 < t_1 \leq 30$ s, insbesondere ausgewählt ist aus dem Bereich $0 < t_1 \leq 15$ s, und insbesondere ausgewählt ist aus dem Bereich $0 < t_1 \leq 2$ s.

**3.** Das Desinfektionsverfahren nach einem der Ansprüche 1 bis 2, wobei der Einwirkzeitraum, der zum Zeitpunkt $t_1$ beginnt und zum Zeitpunkt $t_2$ endet, maximal 90 s, insbesondere maximal 50 s, insbesondere maximal 30 s insbesondere maximal 15 s beträgt.

**4.** Das Desinfektionsverfahren nach einem der Ansprüche 1 bis 3, wobei der pH-Wert der Wirklösung auf der mit Wirklösung kontaktierten Oberfläche im Bereich von Bereich von $2{,}5 \leq pH \leq 5$ liegt, und insbesondere in einem Bereich von $3{,}3 \leq pH \leq 4{,}7$ liegt.

**5.** Das Desinfektionsverfahren nach Anspruch 1 bis 3, wobei der *pH*-Wert der Wirklösung vor Kontakt mit der zu desinfizierenden Oberfläche im Bereich von 2,1 bis 4,5, insbesondere in einem Bereich von 2,1 bis 3,6, und insbesondere in einem Bereich von 2,1 bis 3,2 liegt.

6. Das Desinfektionsverfahren nach einem der Anspruch 1 bis 5, wobei die maximale Ausgangskonzentration an $NO_2^-$ zum Zeitpunkt to eine Konzentration von 200 mM insbesondere von 100 mM nicht überschreitet.

7. Das Desinfektionsverfahren nach Anspruch 1 bis 6, wobei die Effizienz $E = W/W_{max}$ mindestens 10 %, insbesondere mindestens 20 %, insbesondere mindestens 30 % beträgt, wobei

$$W_{\max} = \min(\,[\mathrm{H_2O_2}]_0, [\mathrm{NO_2^-}]_0\,)\ (\exp(-Gt_1) - \exp(-Gt_2))$$

mit $G = \ln\left(\frac{t_2}{t_1}\right)/(t_2 - t_1)$ den bei gegebenen Zeiten $t_2$ und $t_1$ maximal erreichbaren Wirksamkeitsparameter bezeichnet und $\min([H_2O_2]_0,[NO_2]_0)$ die minimale Konzentration ausgewählt aus den initialen Konzentrationen $[H_2O_2]_0$ und $[\mathrm{NO_2^-}]_0$ bezeichnet.

8. Das Desinfektionsverfahren nach Anspruch 1 bis 7, wobei die Ausgangsstoffmengen an Edukten sich um weniger als 10 % voneinander unterscheiden, insbesondere die Ausgangsstoffmenge von $NO_2^-$ um 2 % bis 10 % höher als die Ausgangsstoffmenge von $H_2O_2$ ist.

9. Desinfektionsverfahren nach einem der Ansprüche 1 bis 7, wobei die Ausgangsstoffmengen an Edukten identisch sind.

10. Desinfektionsverfahren nach einem der Ansprüche 1 bis 9, wobei den Edukten und/oder der Wirklösung Zusatzstoffe in Form von Säurepuffern bzw. Säurepufferlösungen zugesetzt werden.

11. Desinfektionsverfahren nach einem der Ansprüche 1 bis 10, wobei eine oder mehrere Plasmaquellen genutzt werden können, um eines oder mehrere der Edukte vor dem Vermischungsschritt herzustellen.

12. Desinfektionsverfahren nach einem der Ansprüche 1 bis 11, wobei das Desinfektionsverfahren nach einem der Ansprüche 1 bis 11 mehrfach angewendet wird.

**Claims**

1. A Disinfection process comprising at least the reactants $H_2O_2$ and $NO_2^-$ consisting of several substeps comprising at least:

- a mixing step, wherein the reactants are mixed to obtain an active solution;
- a distribution step in which the active solution is distributed on a surface to be disinfected,

wherein the mixing step and the distribution step take place in a processing period $Z_A$ which starts at the time $t_0$ when the reactants are first brought into contact with each other and ends at the time $t_1$, at which every point on the surface to be disinfected is wetted with active solution, wherein $t_0$ is 0 and $t_1$ is higher than $t_0$, and

- followed by an action step in which the distributed active solution acts on the surface contacted with active solution over an action period $Z_e$, which begins at the time $t_1$ and ends after the time period $Z_e$ at the time $t_2$,

wherein $t_2$ represents the time at which each point on the surface contacted with active solution is wetted with active solution for a sufficient time to obtain a disinfecting effect, and wherein $t_2$ is higher than $t_1$,
**characterized in that**
the maximum $NO_2^-$ concentration $[NO_2^-]_0$ at time $t_0$ of the mixing step is 300 mM, the time-dependent concentrations of the reactants $[H_2O_2]$ and $[NO_2^-]$ during the processing period and the action period are determined by

$$[NO_2^-] = \frac{A}{k_1} \text{ and } [H_2O_2] = \frac{A+D}{k_1 + rk_1},$$

wherein

$$A = -\frac{D}{1-exp(D(t-C))},\ C = -\frac{ln\left(\frac{D}{[NO_2^-]_0 \cdot k_1}+1\right)}{D},\ D = [H_2O_2]_0 \cdot (k_1 + rk_1) - [NO_2^-]_0 \cdot k_1,$$

$$k_1 = k_4 \frac{[H_3O^+]^2}{\left(K_{S,H_3O^+} + [H_3O^+]\right)\left(K_{S,HNO_2} + [H_3O^+]\right)}, k_4 = 3.56 \cdot 10^{14} exp\left(-\frac{E_A}{RT}\right) M^{-1}s^{-1},$$

$$K_{S,HNO_2} = 5.13 \cdot 10^{-4}, K_{S,H_3O^+} = 2 \cdot 10^{-2}, [H_3O^+] = 10^{-pH}, E_A = 70\ kJ/mol$$

and $[H_2O_2]_0$ denotes the initial $H_2O_2$ concentration at time $t_0$ of the mixing step and wherein r = 0.11 is to be set, and the time-integrated reaction rate W over the active period $Z_e$ fulfills the inequality

$$W = \int_{t_1}^{t_2} k_1 \cdot [H_2O_2] \cdot [NO_2^-] dt \geq 10mM$$

wherein $t_2$ does not exceed 3 minutes, and
wherein $k_1$ denotes the rate constant of the reaction between $H_2O_2$ and $NO_2^-$,
wherein $k_1$ depends on the pH value of the active solution on the surface and
wherein the pH value of the active solution on the surface and the temperature may exhibit a time dependence, and
wherein the pH value of the active solution before contact with the surface to be disinfected and the pH value of the active solution on the surface contacted with active solution are in the range of $2.1 \leq pH \leq 6.8$.

**2.** The Disinfection process according to claim 1, wherein the processing period, that ends at time $t_1$ is selected from the range $0 < t_1 \leq 75$ s, in particular is selected from the range $0 < t_1 \leq 30$ s, in particular is selected from the range $0 < t_1 \leq 15$ s, and in particular is selected from the range $0 < t_1 \leq 2$ s.

**3.** The Disinfection process according to any one of claims 1 to 2, wherein the action period, which begins at time $t_1$ and ends at time $t_2$, is a maximum of 90 s, in particular a maximum of 50 s, in particular a maximum of 30 s, in particular a maximum of 15 s.

**4.** The Disinfection process according to any one of claims 1 to 3, wherein the pH value of the active solution on the surface contacted with active solution is in the range of $2.5 \leq pH \leq 5$, and in particular in a range of $3.3 \leq pH \leq 4.7$.

**5.** The Disinfection process according to claim 1 to 3, wherein the pH value of the active solution before contact with the surface to be disinfected is in the range from 2.1 to 4.5, in particular in the range from 2.1 to 3.6, and in particular in the range from 2.1 to 3.2.

**6.** The Disinfection process according to any one of claims 1 to 5, wherein the maximum initial concentration of $NO_2^-$ at time $t_0$ does not exceed a concentration of 200 mM, in particular 100 mM.

**7.** The Disinfection process according to claim 1 to 6, wherein the efficiency $E = W/W_{max}$ is at least 10%, in particular at least 20%, in particular at least 30%, wherein

$$W_{max} = min([H_2O_2]_0, [NO_2^-]_0)\left(exp(-Gt_1) - exp(-Gt_2)\right)$$

wherein $G = \ln\left(\frac{t_2}{t_1}\right)/(t_2 - t_1)$ denotes for the given times $t_2$ and $t_1$ the maximum achievable effectiveness parameter and $min([H_2O_2]_0, [NO_2]_0)$ denotes the minimum concentration selected from the initial concentrations

$[H_2O_2]_0$ and $[NO_2^-]_0$.

**8.** The Disinfection process according to claims 1 to 7, wherein the starting amounts of reactants differ from each other by less than 10%, in particular the starting amount of $NO_2^-$ is 2% to 10% higher than the starting amount of $H_2O_2$.

**9.** The Disinfection process according to any one of claims 1 to 7, wherein the starting amounts of reactants are identical.

**10.** The Disinfection process according to any one of claims 1 to 9, wherein additives in the form of acid buffers or acid buffer solutions are added to the reactants and/or the active solution.

**11.** The Disinfection process according to any one of claims 1 to 10, wherein one or more plasma sources may be used to produce one or more of the reactants prior to the mixing step.

**12.** The Disinfection process according to any one of claims 1 to 11, wherein the Disinfection process according to any one of claims 1 to 11 is applied several times.

## Revendications

**1.** Procédé de désinfection présentant au moins les matériaux de départ $H_2O_2$ et $NO_2^-$ constitué de plusieurs étapes partielles comprenant au moins :

- une étape de mélange, dans lequel les matériaux de départ sont mélangés pour l'obtention d'une solution active ;
- une étape d'étalement lors de laquelle la solution active est étalée sur une surface à désinfecter,

dans lequel l'étape de mélange et l'étape d'étalement ont lieu dans une période de traitement $Z_A$ qui commence à l'instant $t_0$ lorsque les matériaux de départ sont d'abord amenés en contact l'un avec l'autre et se termine à l'instant $t_1$ auquel chaque point sur la surface à désinfecter est mouillé avec la solution active, dans lequel $t_0$ est égal à 0 et $t_1$ est supérieur à $t_0$, et

- ensuite une étape d'action lors de laquelle la solution active étalée agit sur la surface mise en contact avec la solution active sur une période d'action $Z_e$ qui commence à l'instant $t_1$ et se termine à l'instant $t_2$ après l'intervalle de temps $Z_e$,

dans lequel $t_2$ représente l'instant auquel chaque point sur la surface mise en contact avec la solution active est mouillé suffisamment longtemps avec la solution active pour obtenir une action désinfectante, et dans lequel $t_2$ est supérieur à $t_1$,

**caractérisé en ce que**

la concentration maximale en $NO_2^-[NO_2^-]_0$ à l'instant $t_0$ de l'étape de mélange se situe à 300 mM, les concentrations à dépendance temporelle des matériaux de départ $[H_2O_2]$ et $[NO_2^-]$ sont données pendant la période de traitement et la période d'action par

$$[NO_2^-] = \frac{A}{k_1} \quad \text{et} \quad [H_2O_2] = \frac{A+D}{k_1 + rk_1},$$

dans lesquelles

$$A = -\frac{D}{1-\exp(D(t-C))}, \quad C = -\frac{\ln\left(\frac{D}{[NO_2^-]_0 \cdot k_1} + 1\right)}{D}, \quad D = [H_2O_2]_0 \cdot (k_1 + rk_1) - [NO_2^-]_0 \cdot k_1,$$

$$k_1 = k_4 \frac{[H_3O^+]^2}{\left(K_{S,H_3O_2^+} + [H_3O^+]\right)\left(K_{S,HNO_2} + [H_3O^+]\right)}, \quad k_4 = 3.56 \cdot 10^{14} \exp\left(-\frac{E_A}{RT}\right) M^{-1}s^{-1},$$

$$K_{S,HNO_2} = 5{,}13 \times 10^{-4},\ K_{S,H_3O_2^+} = 2 \times 10^{-2},\ [H_3O^+] = 10^{-pH},\ E_A = 70\ \mathrm{kJ/mol}$$

et $[H_2O_2]_0$ désigne la concentration initiale en $H_2O_2$ à l'instant $t_0$ de l'étape de mélange, et dans lequel r = 0,11 est à placer, et le taux de réaction intégré au temps W sur la période d'action $Z_e$ satisfait l'inéquation

$$W = \int_{t_1}^{t_2} k_1 \cdot [H_2O_2] \cdot [NO_2^-]\, dt \geq 10\ mM$$

dans laquelle $t_2$ ne dépasse pas 3 minutes, et dans laquelle $k_1$ désigne la constante de vitesse de la réaction entre $H_2O_2$ et $NO_2^-$, dans laquelle $k_1$ dépend de la valeur de pH de la solution active sur la surface et dans laquelle la valeur de pH de la solution active sur la surface et la température peuvent présenter une dépendance temporelle, et dans laquelle la valeur de pH de la solution active avant contact avec la surface à désinfecter et la valeur de pH de la solution active sur la surface mise en contact avec la solution active se situent dans la région de $2{,}1 \leq pH < 6{,}8$.

**2.** Procédé de désinfection selon la revendication 1, dans lequel la période de traitement qui se termine à l'instant $t_1$ est sélectionnée parmi la région $0 < t_1 \leq 75$ s, notamment est sélectionnée parmi la région $0 < t_1 \leq 30$ s, notamment est sélectionnée parmi la région $0 < t_1 \leq 15$ s, et notamment est sélectionnée parmi la région $0 < t_1 \leq 2$ s.

**3.** Procédé de désinfection selon une des revendications 1 à 2, dans lequel la période d'action qui commence à l'instant $t_1$ et se termine à l'instant $t_2$ se monte au maximum à 90 s, notamment au maximum à 50 s, notamment au maximum à 30 s, notamment au maximum à 15 s.

**4.** Procédé de désinfection selon une des revendications 1 à 3, dans lequel la valeur de pH de la solution active sur la surface mise en contact avec la solution active se situe dans la région de $2{,}5 \leq pH \leq 5$, et notamment dans une région de $3{,}3 \leq pH \leq 4{,}7$.

**5.** Procédé de désinfection selon les revendications 1 à 3, dans lequel la valeur de pH de la solution active avant contact avec la surface à désinfecter se situe dans la région de 2,1 à 4,5, notamment dans une région de 2,1 à 3,6, et notamment dans une région de 2,1 à 3,2.

**6.** Procédé de désinfection selon une des revendications 1 à 5, dans lequel la concentration de départ maximale en $NO_2^-$ à l'instant $t_0$ ne dépasse pas une concentration de 200 mM, notamment de 100 mM.

**7.** Procédé de désinfection selon les revendications 1 à 6, dans lequel l'efficacité $E = W/W_{max}$ se monte à au moins 10 %, notamment au moins 20%, notamment au moins 30 %, dans lequel

$$G = \ln\left(\frac{t_2}{t_1}\right) / (t_2 - t_1)$$

$W_{max}=\min([H_2O_2]_0,[NO_2^-]_0)\ (\exp(-Gt_1)-\exp(-Gt_2))$ avec qui désigne le paramètre d'efficacité pouvant être atteint au maximum aux temps donnés $t_2$ et $t_1$ et $\min([H_2O_2]_0,[NO_2^-]_0)$ désigne la concentration minimale sélectionnée parmi les concentrations initiales $[H_2O_2]_0$ et $[NO_2^-]_0$.

**8.** Procédé de désinfection selon les revendications 1 à 7, dans lequel les quantités de substance de départ en matériaux de départ se différencient l'une de l'autre de moins de 10 %, notamment la quantité de substance de départ de $NO_2^-$ est de 2 % à 10 % supérieure à la quantité de substance de départ de $H_2O_2$.

**9.** Procédé de désinfection selon une des revendications 1 à 7, dans lequel les quantités de substance de départ en matériaux de départ sont identiques.

**10.** Procédé de désinfection selon une des revendications 1 à 9, dans lequel des additifs sont ajoutés sous forme de tampons acides ou solutions de tampon acide aux matériaux de départ et/ou à la solution active.

**11.** Procédé de désinfection selon une des revendications 1 à 10, dans lequel une ou plusieurs sources de plasma peuvent être utilisées pour fabriquer un ou plusieurs des matériaux de départ avant l'étape de mélange.

**12.** Procédé de désinfection selon une des revendications 1 à 11, dans lequel le procédé de désinfection selon une des revendications 1 à 11 est employé plusieurs fois.

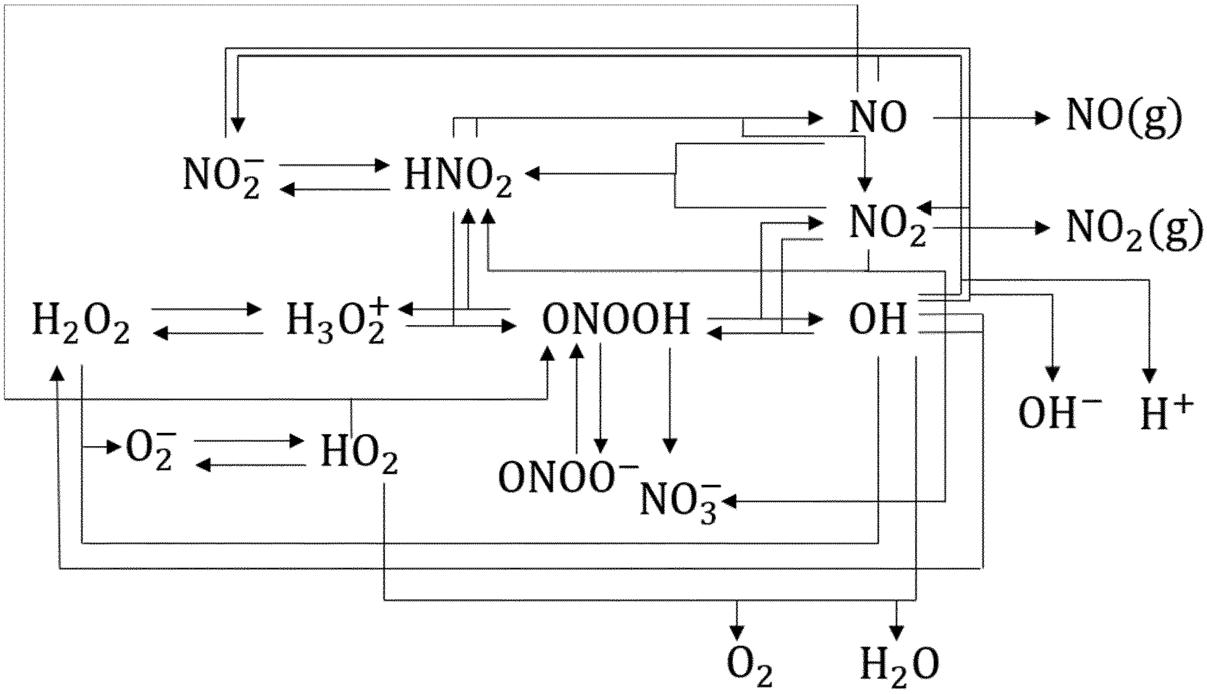

Fig. 1

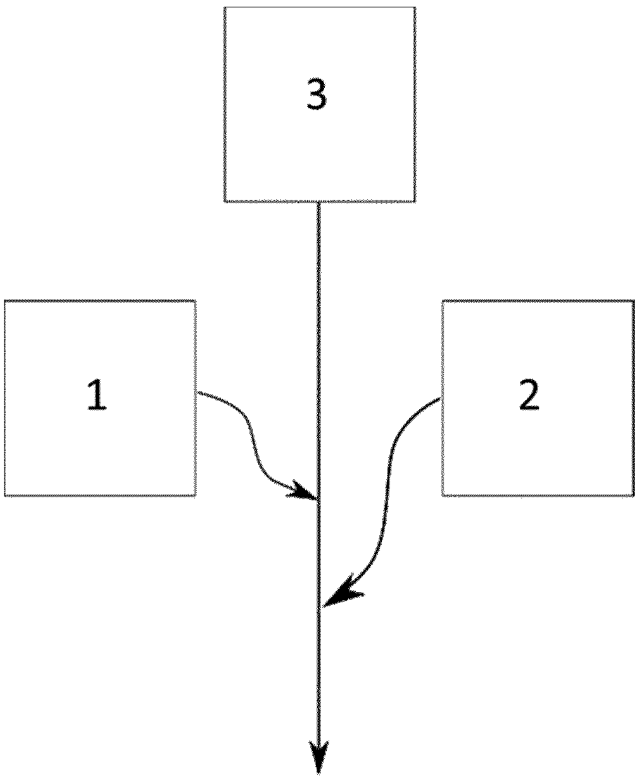


Fig. 2

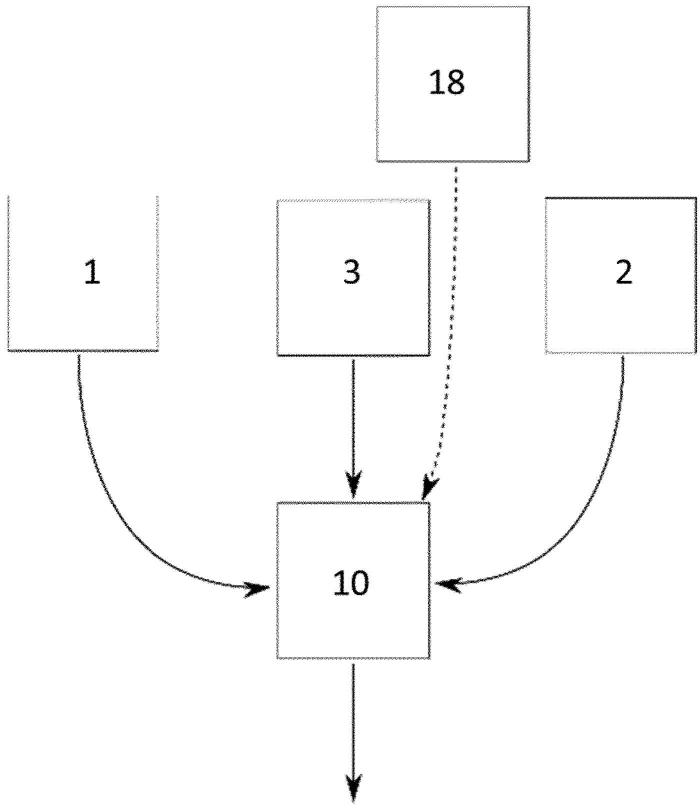


Fig. 3

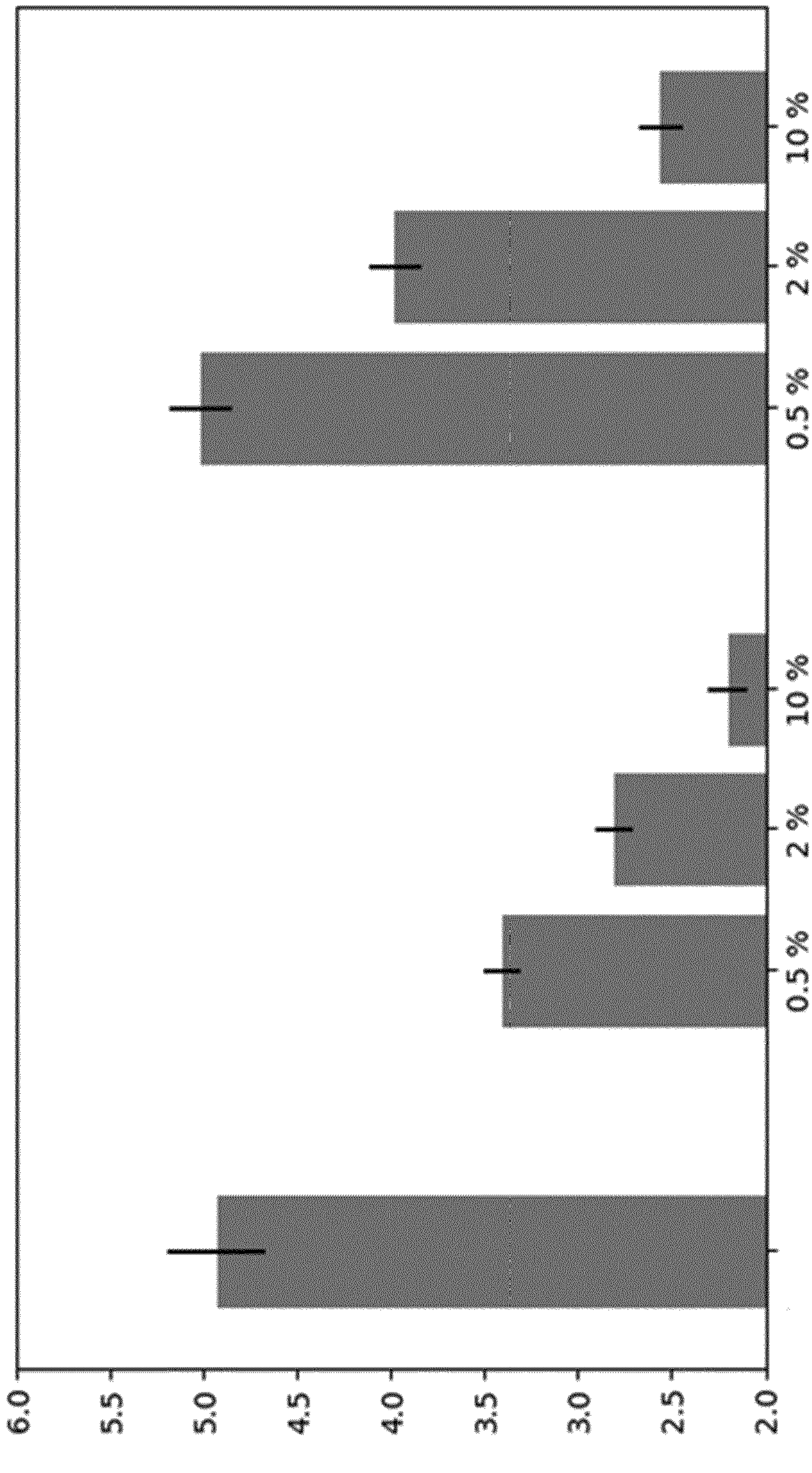


Fig. 4

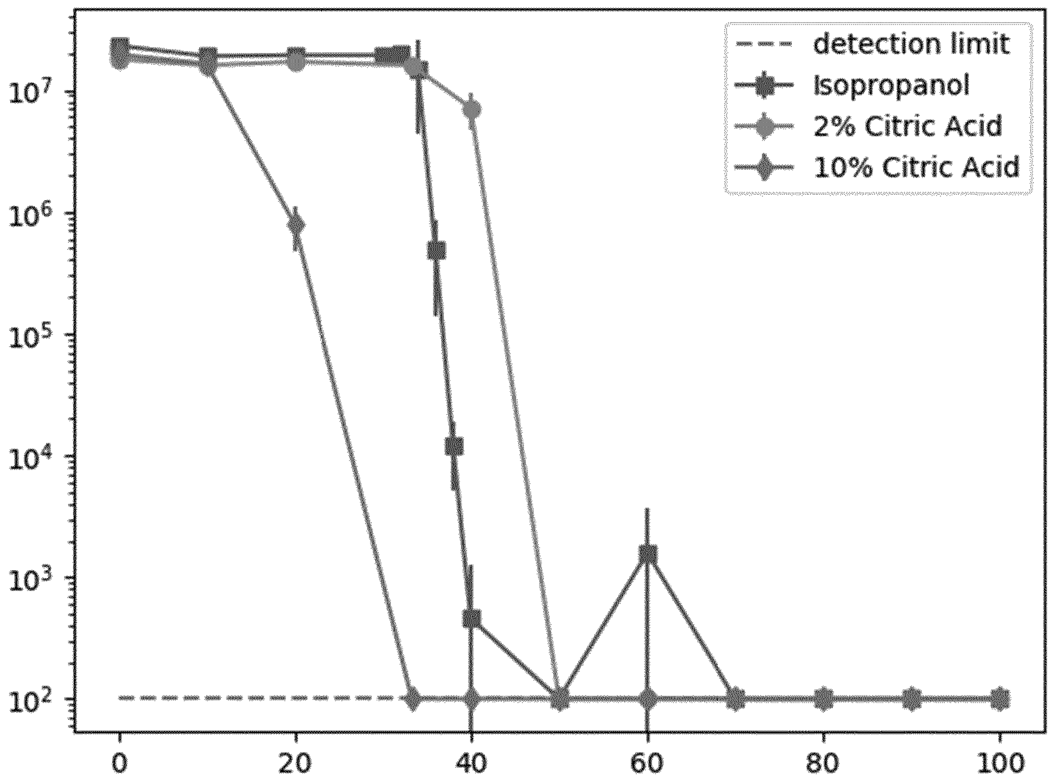

detection limit
Isopropanol
2% Citric Acid
10% Citric Acid
$10^7$
$10^6$
$10^5$
$10^4$
$10^3$
$10^2$
0
20
40
60
80
100


Fig. 5

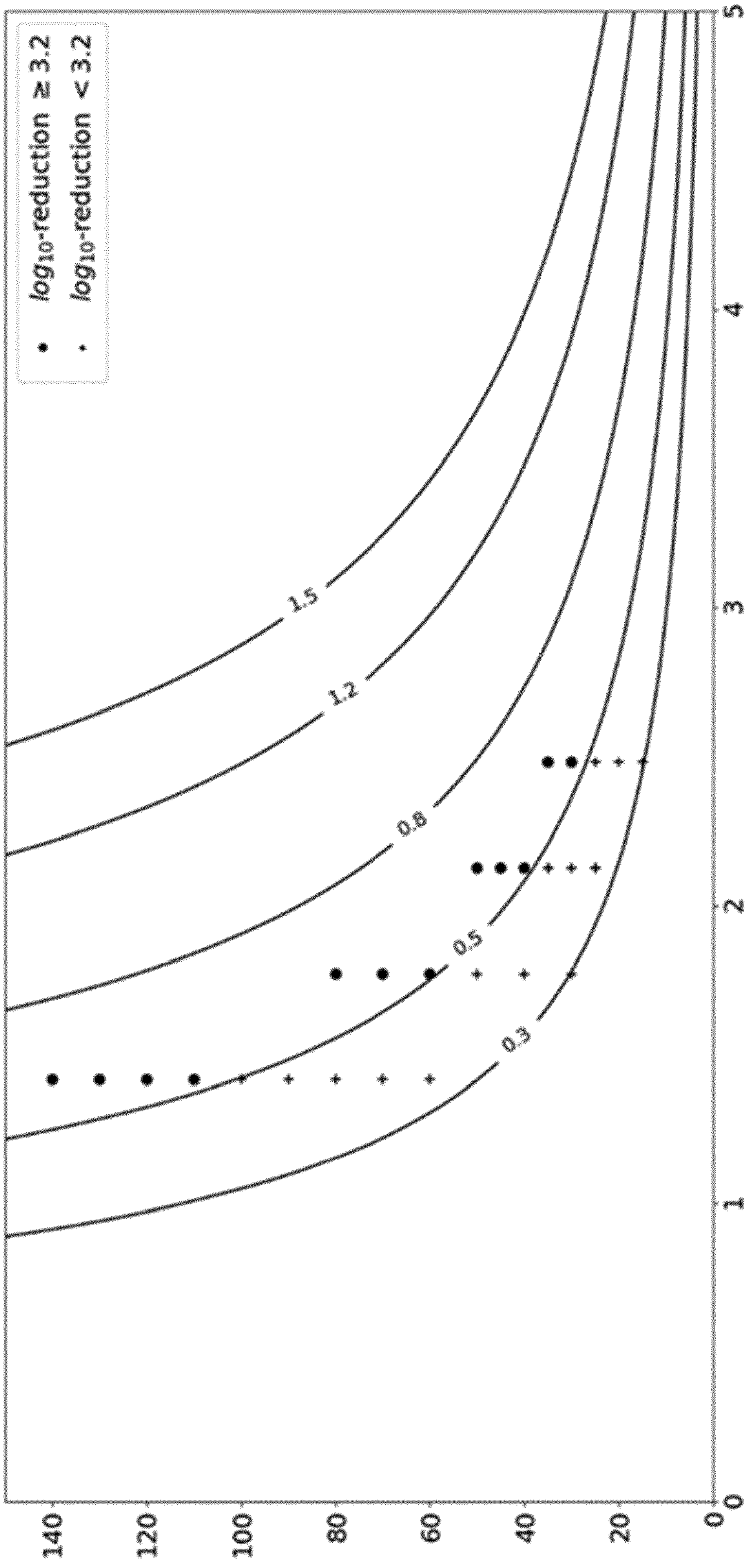

$log_{10}$-reduction ≥ 3.2
$log_{10}$-reduction < 3.2
1.5
1.2
0.8
0.5
0.3
0
1
2
3
4
5
140
120
100
80
60
40
20
0


Fig. 6

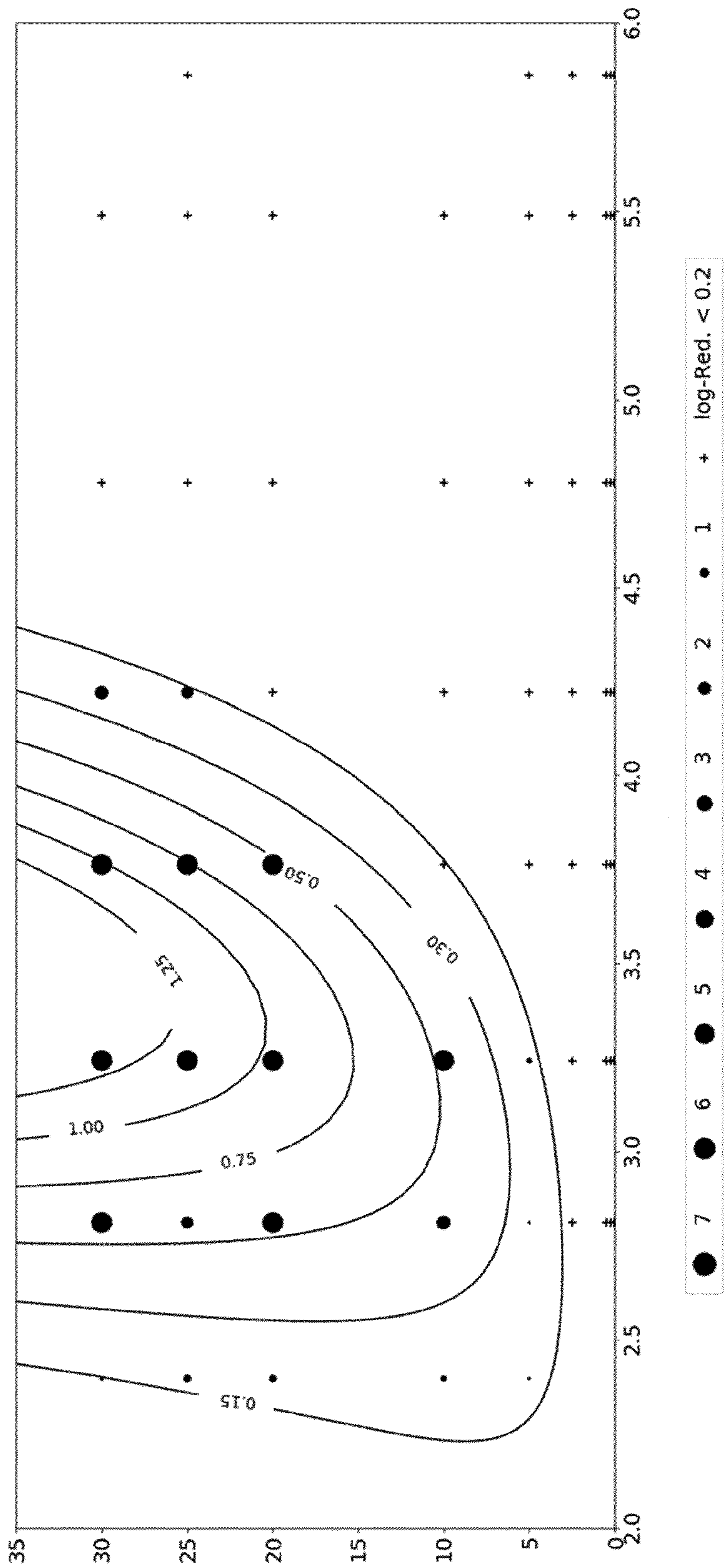

2.0
2.5
3.0
3.5
4.0
4.5
5.0
5.5
6.0
0
5
10
15
20
25
30
35
0.15
0.30
0.50
0.75
1.00
1.25
7
6
5
4
3
2
1
log-Red. < 0.2


Fig. 7

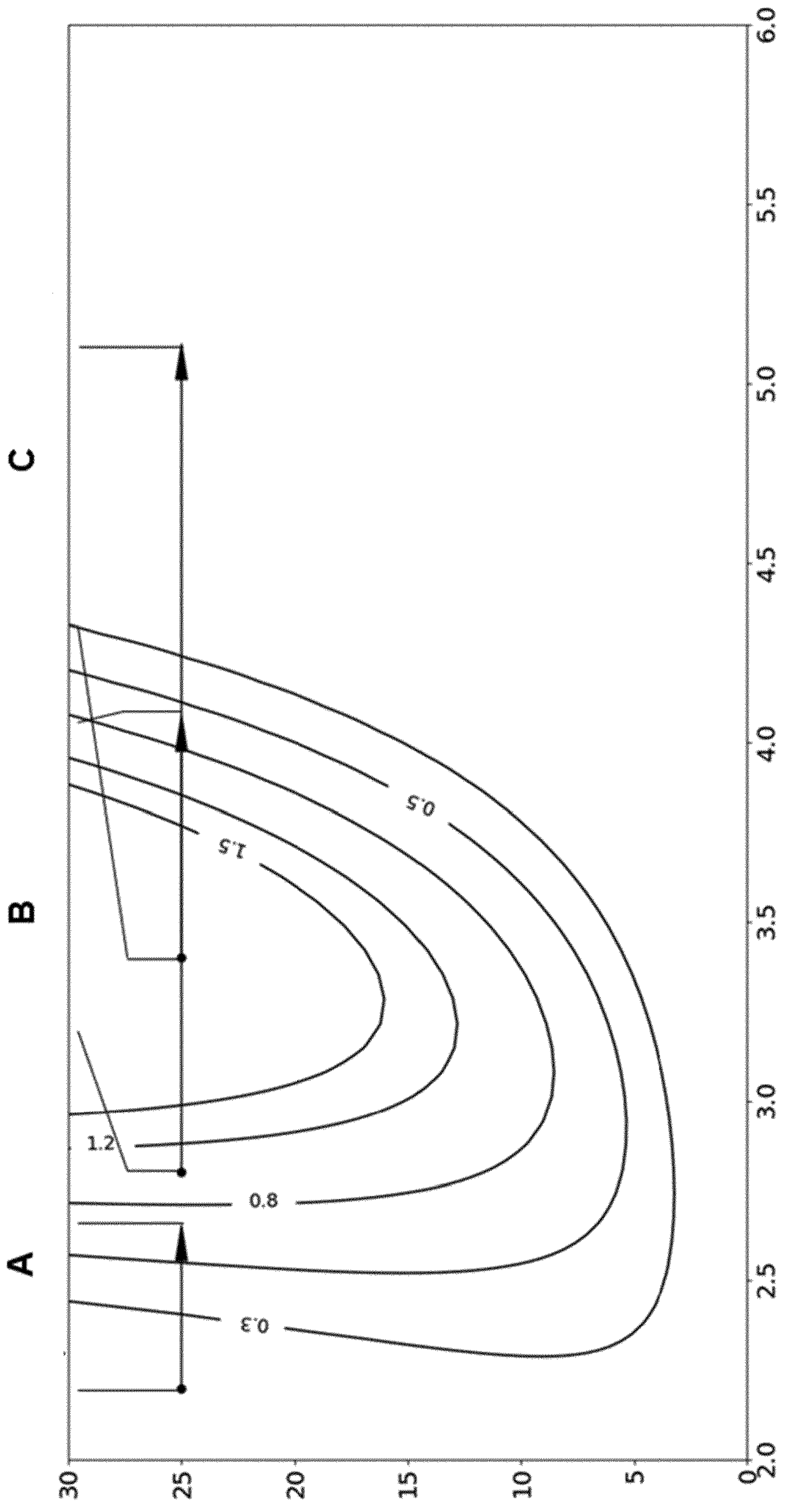


Fig. 8

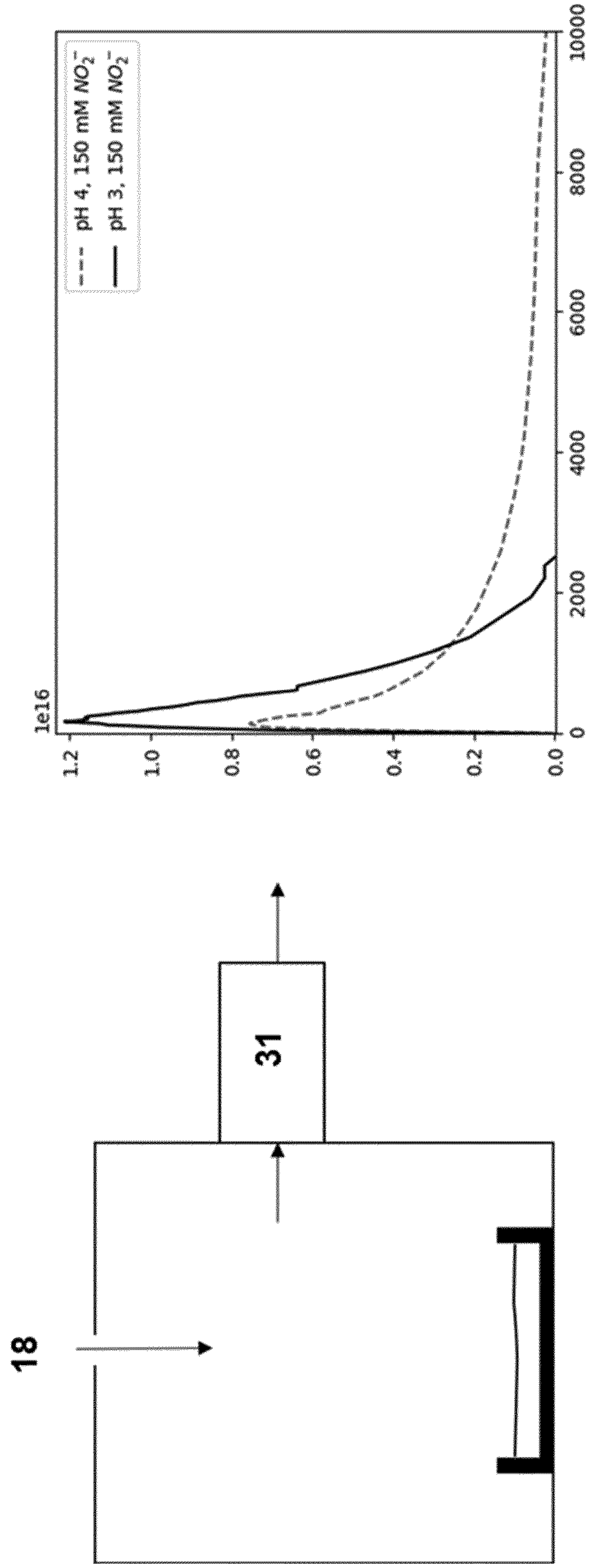
pH 4, 150 mM NO2−
pH 3, 150 mM NO2−
1e16
1.2
1.0
0.8
0.6
0.4
0.2
0.0
0
2000
4000
6000
8000
10000
18
31

Fig. 9

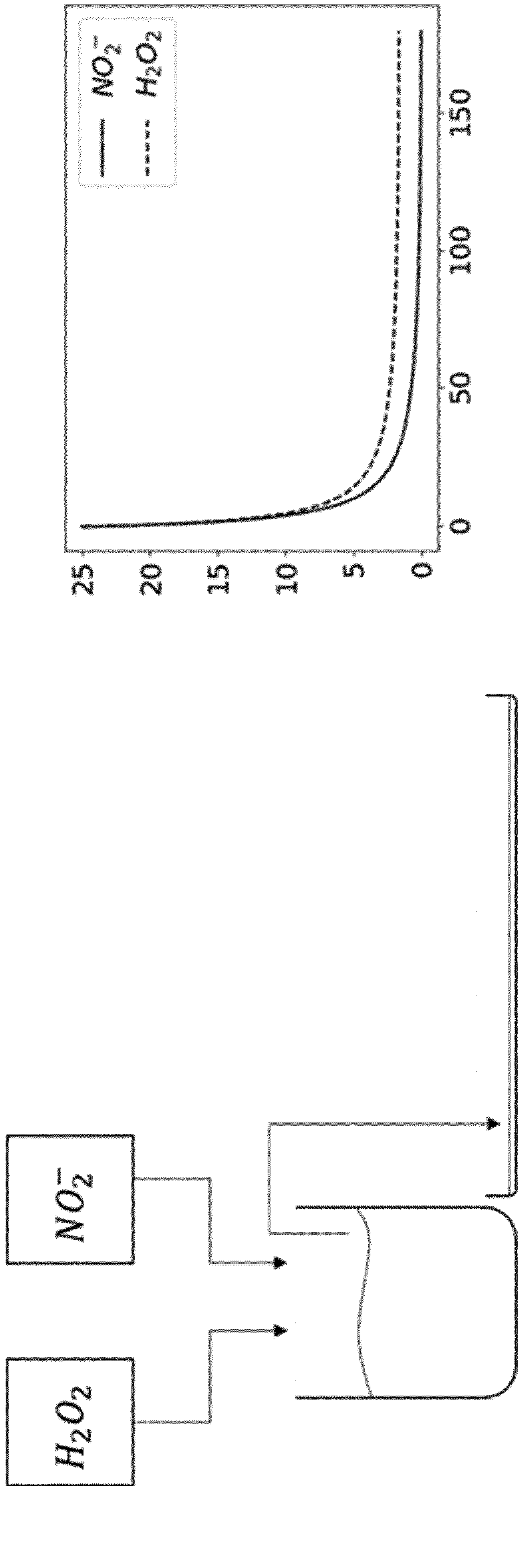


Fig. 10

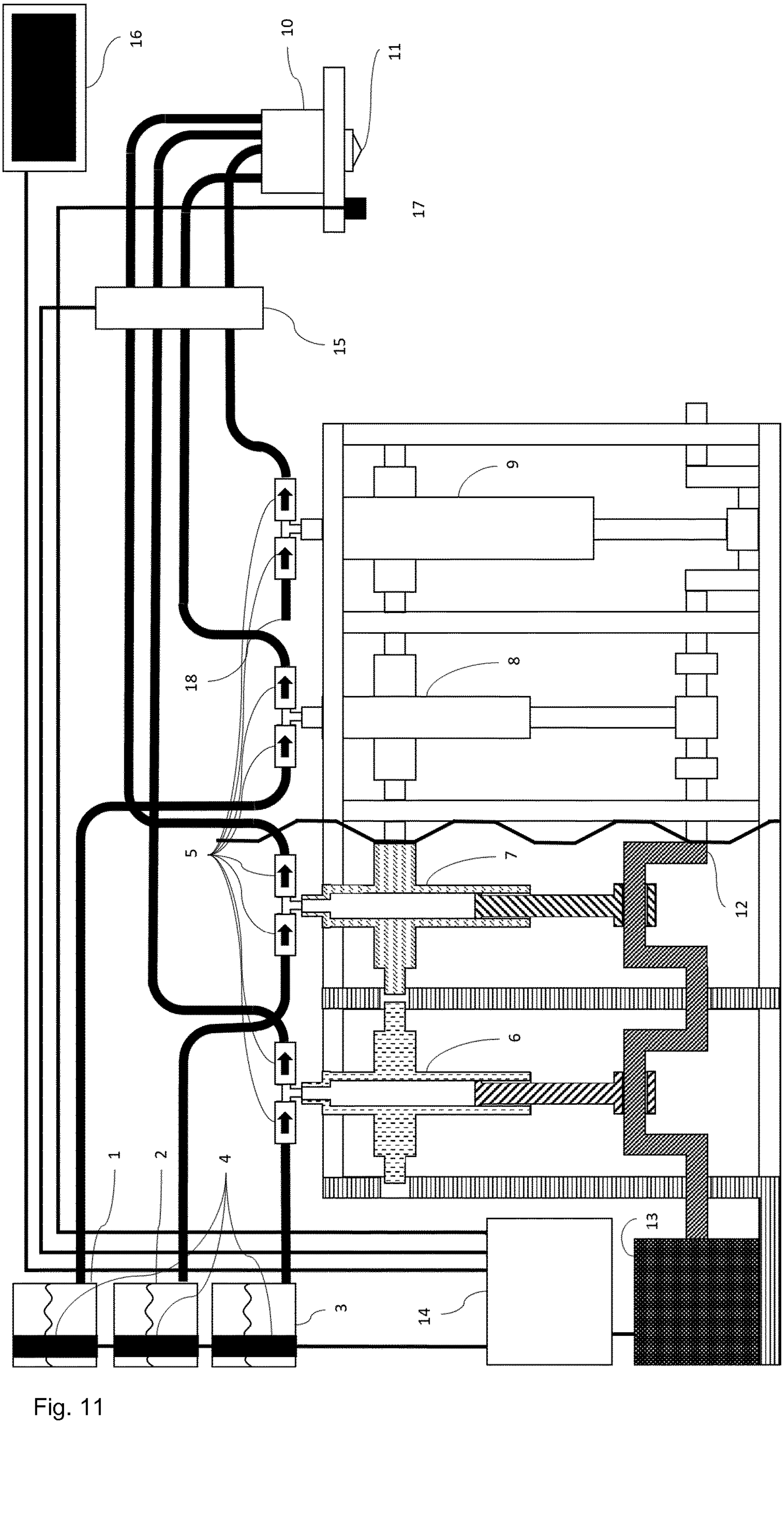


Fig. 11

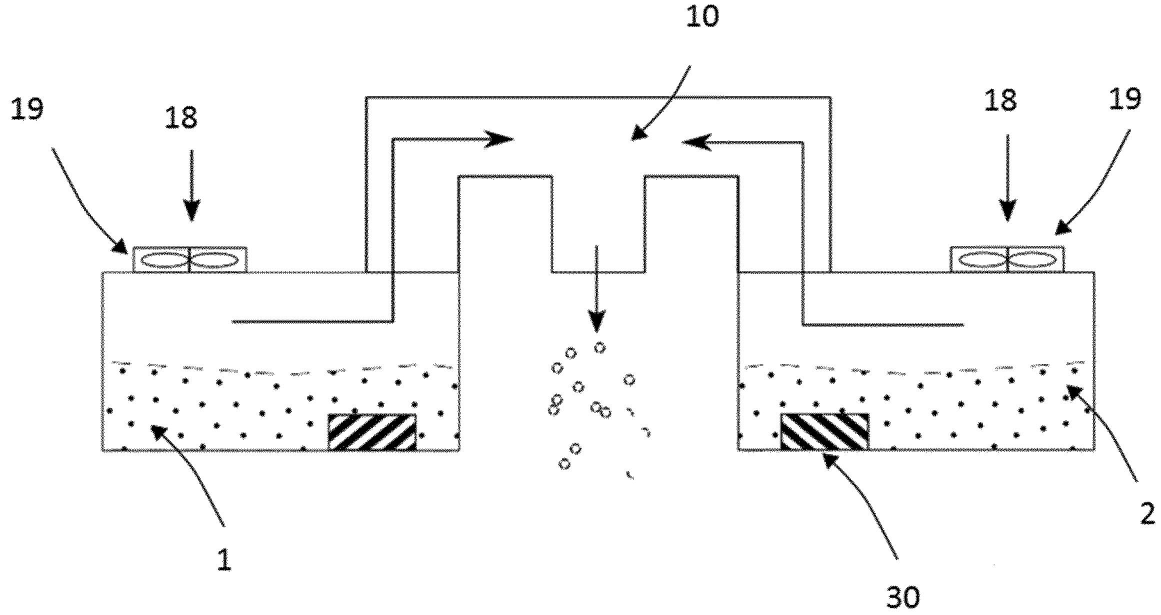


Fig. 12

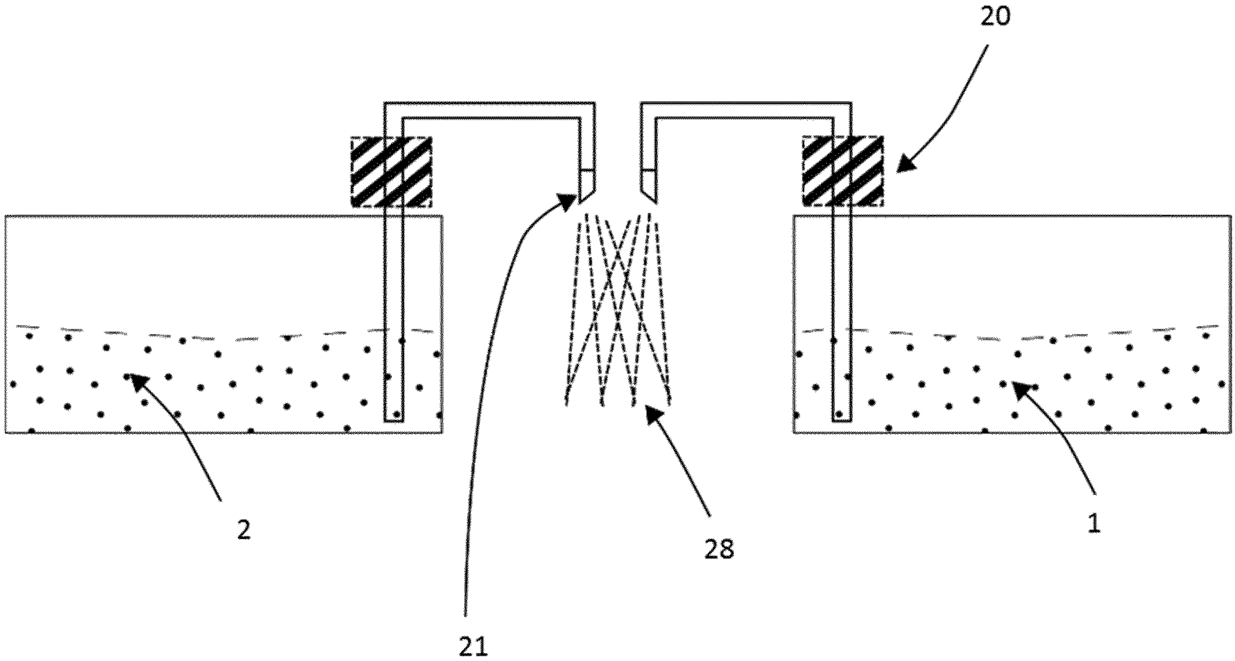


Fig. 13

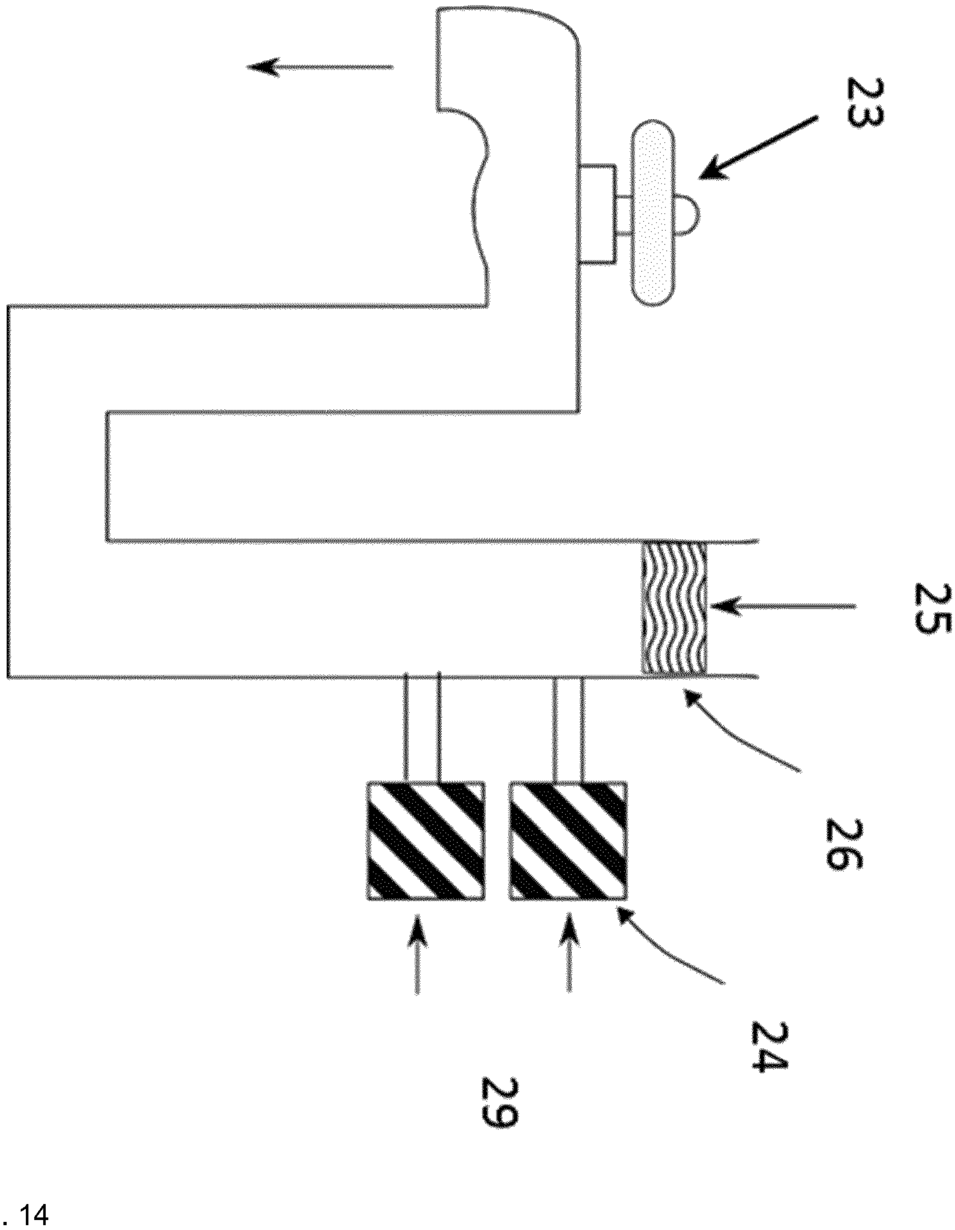


Fig. 14

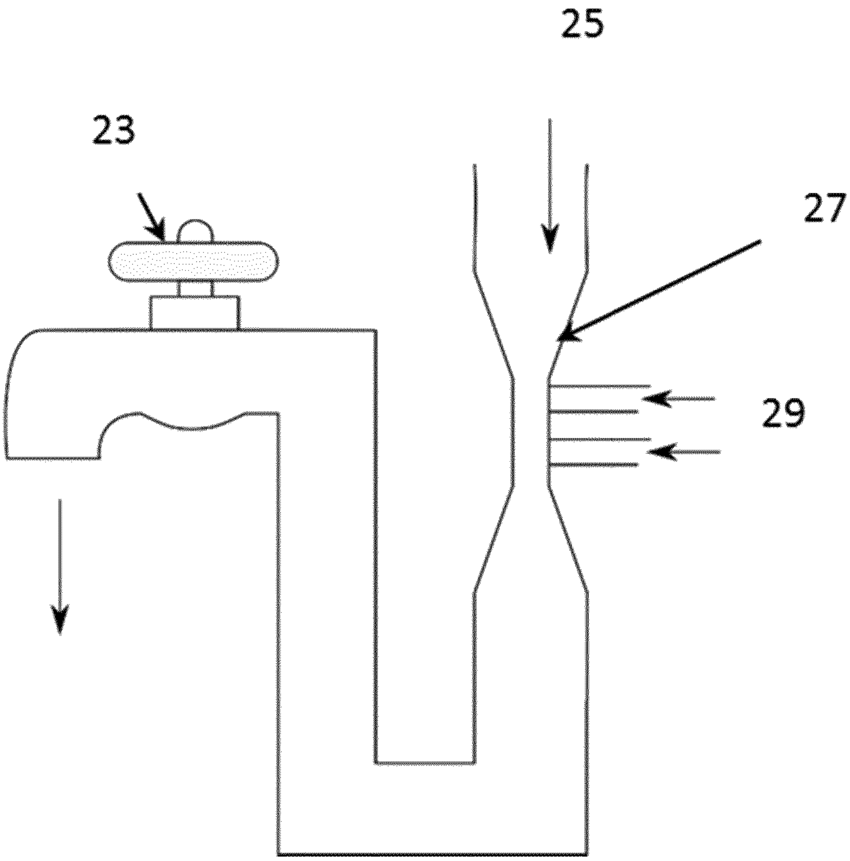


Fig. 15

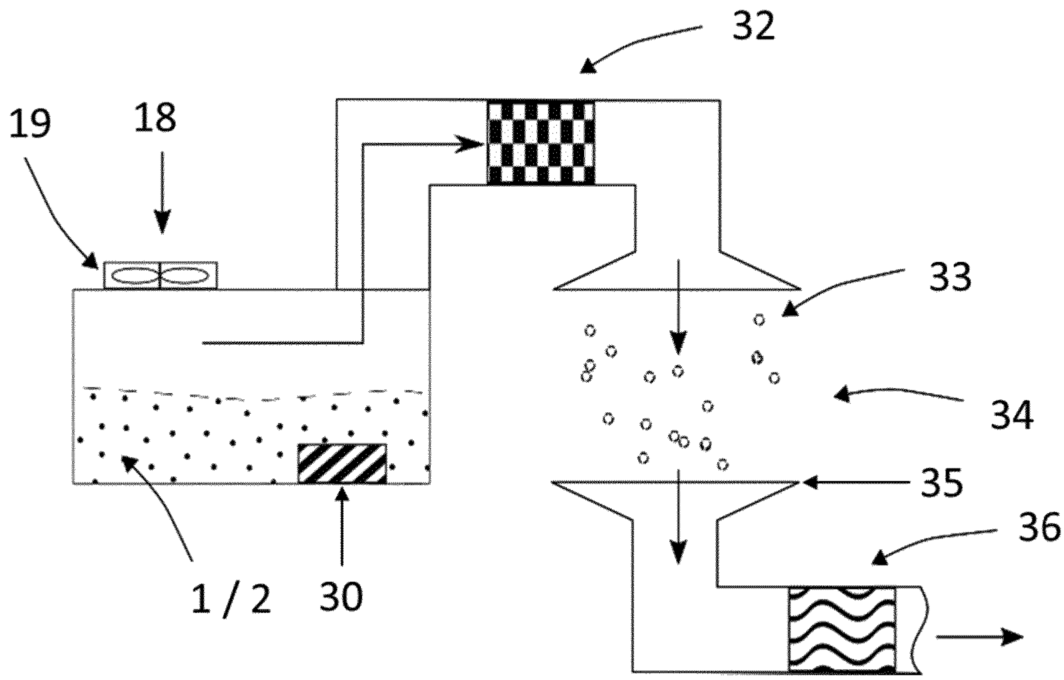


Fig. 16

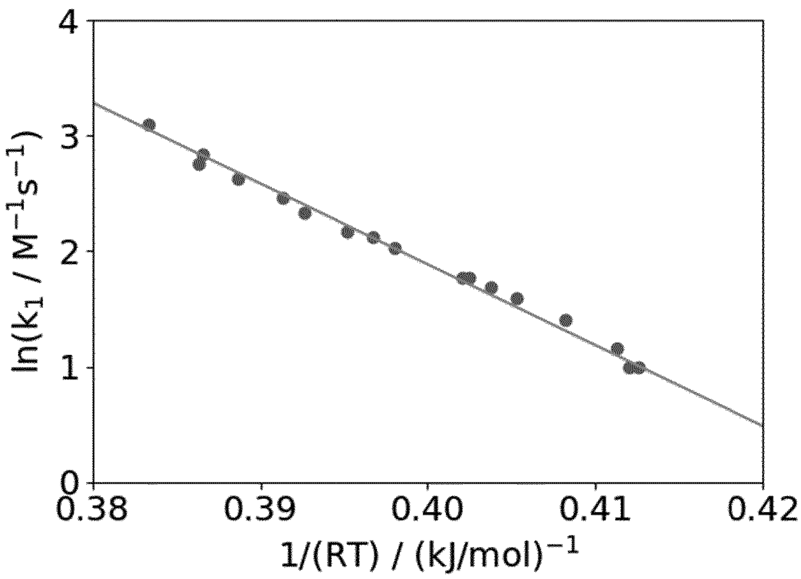
4
3
2
1
0
0.38
0.39
0.40
0.41
0.42
ln(k1 / M−1s−1)
1/(RT) / (kJ/mol)−1

Fig. 17

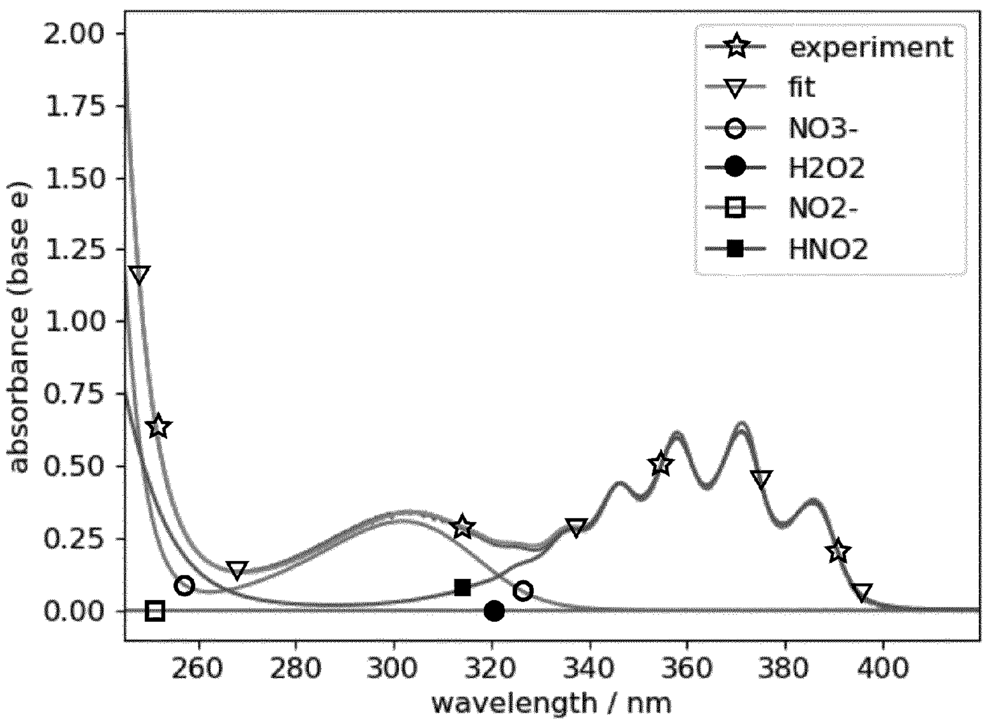
experiment
fit
NO3-
H2O2
NO2-
HNO2
2.00
1.75
1.50
1.25
1.00
0.75
0.50
0.25
0.00
260
280
300
320
340
360
380
400
absorbance (base e)
wavelength / nm

Fig. 18

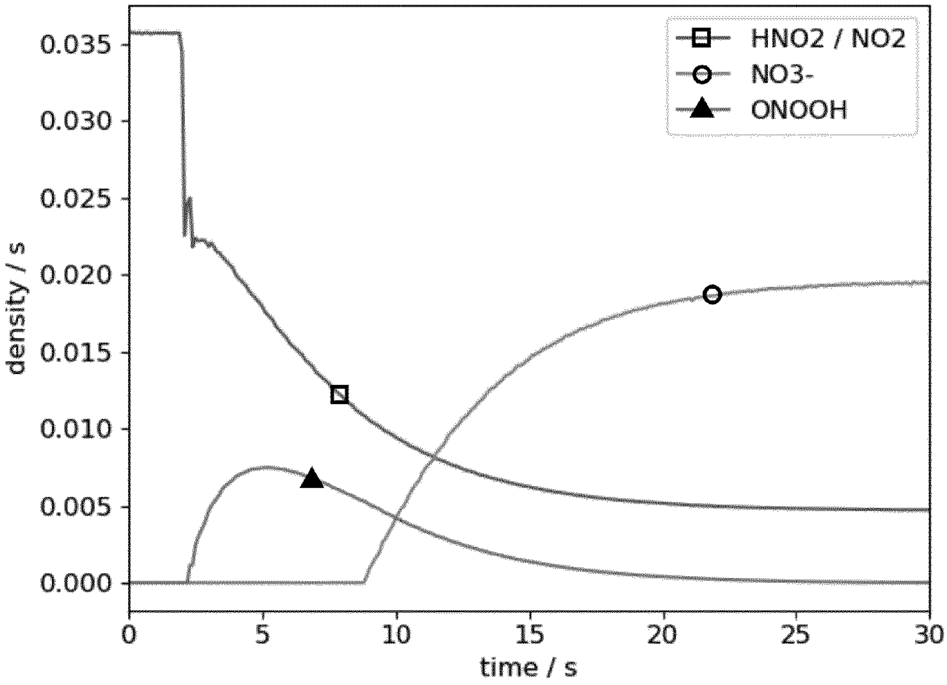
HNO2 / NO2
NO3-
ONOOH
0.035
0.030
0.025
0.020
0.015
0.010
0.005
0.000
density / s
0
5
10
15
20
25
30
time / s

Fig. 19

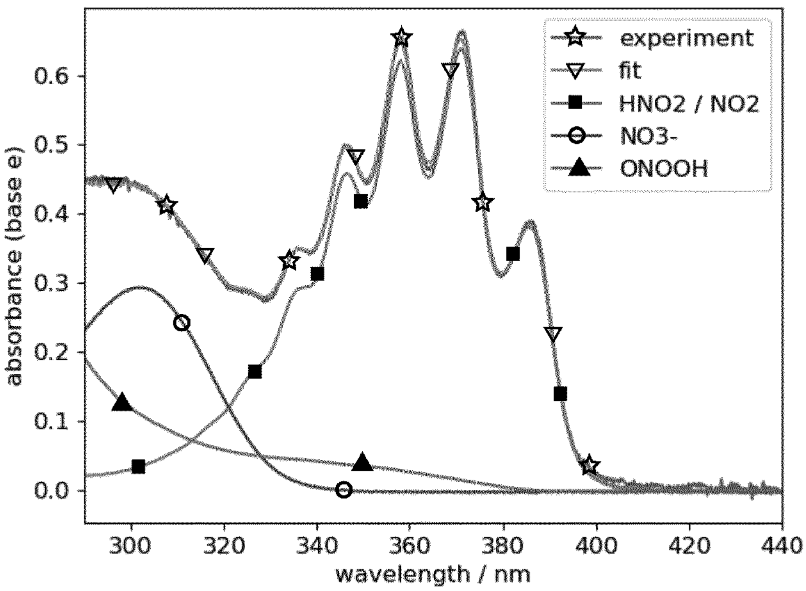
experiment
fit
HNO2 / NO2
NO3-
ONOOH
0.6
0.5
0.4
0.3
0.2
0.1
0.0
absorbance (base e)
300
320
340
360
380
400
420
440
wavelength / nm

Fig. 20

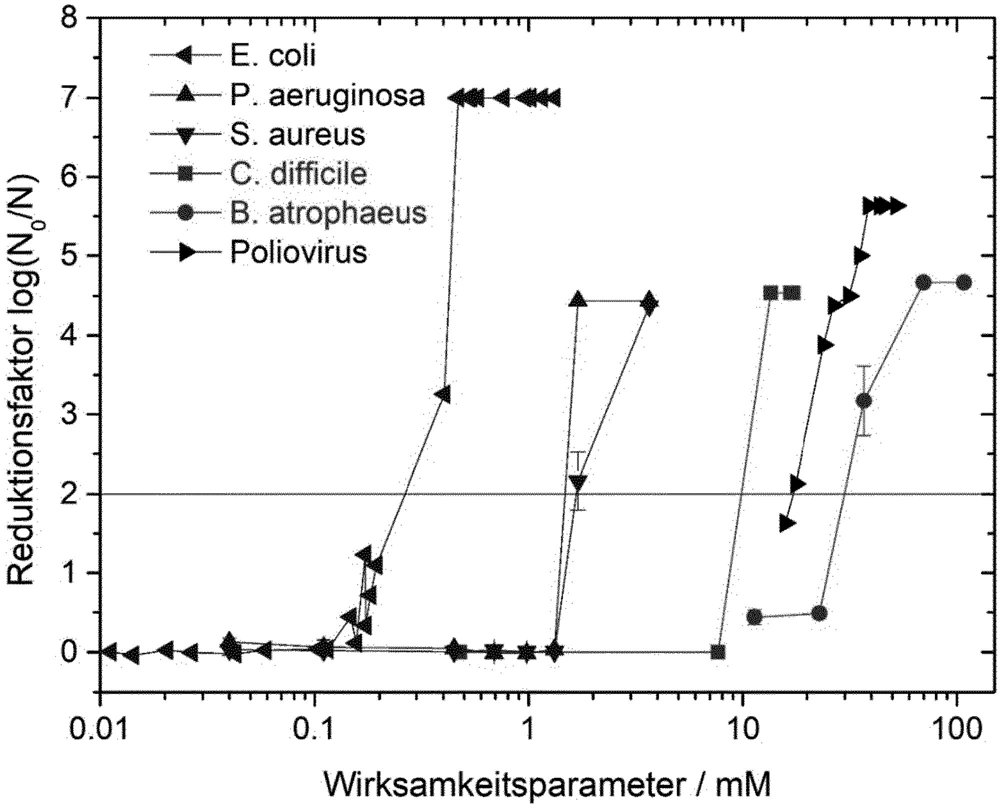

E. coli
P. aeruginosa
S. aureus
C. difficile
B. atrophaeus
Poliovirus
Reduktionsfaktor log($N_0$/N)
Wirksamkeitsparameter / mM
0.01
0.1
1
10
100
8
7
6
5
4
3
2
1
0


Fig. 21

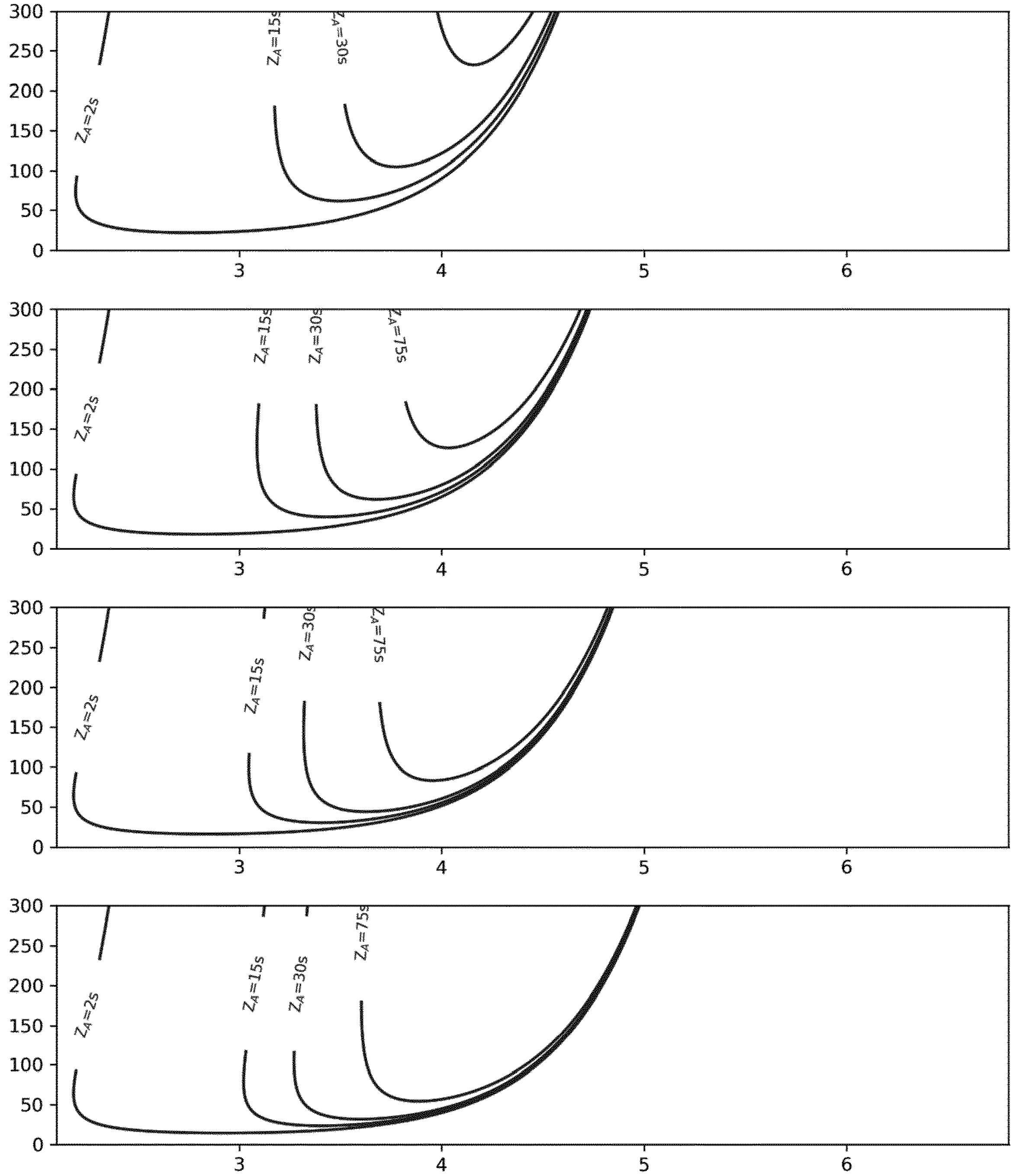
300
250
200
150
100
50
0
3
4
5
6
$Z_A$=2s
$Z_A$=15s
$Z_A$=30s
300
250
200
150
100
50
0
3
4
5
6
$Z_A$=2s
$Z_A$=15s
$Z_A$=30s
$Z_A$=75s
300
250
200
150
100
50
0
3
4
5
6
$Z_A$=2s
$Z_A$=15s
$Z_A$=30s
$Z_A$=75s
300
250
200
150
100
50
0
3
4
5
6
$Z_A$=2s
$Z_A$=15s
$Z_A$=30s
$Z_A$=75s

Fig. 22

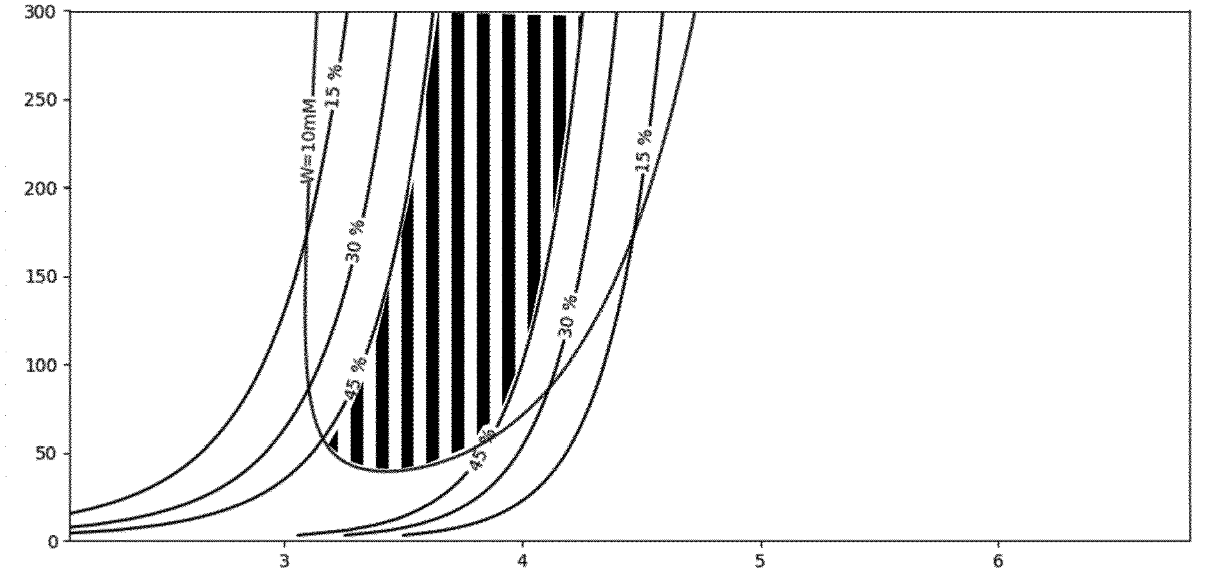


Fig. 23

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2011134010 A1
- US 8871146 B, Ikawa
- US 20170172149 A, Kitano
- EP 17150571, Lukes
- EP 1715057 A
- WO 2011134010 A

### In der Beschreibung aufgeführte Nicht-Patentliteratur


- **ANBAR, M. ; TAUBE, H.** Interaction of Nitrous Acid with Hydrogen Peroxide and with Water. *Journal of the American Chemical Society,* 1954, vol. 76 (24), 6243-6247, https://doi. org/10.1021 /ja01653a007
- **DAMSCHEN, D. E. ; MARTIN, L. R.** Aqueous aerosol oxidation of nitrous acid by O2, O3AND H2O2. *Atmospheric Environment,* 1967, vol. 17 (10), 2005-2011, https://doi.org/10.1016/0004-6981(83)90357-8
- **E. HALFPENNY, P. L. R.** Pernitrous acid. The reaction between hydrogen peroxide and nitrous acid, and the properties of an intermediate product. *Journal of the Chemical Society,* 1952, 928-938
- **HEASELGRAVE, W. ; ANDREW, P. W. ; KILVINGTON, S.** Acidified nitrite enhances hydrogen peroxide disinfection of Acanthamoeba, bacteria and fungi. *Journal of Antimicrobial Chemotherapy,* 2010, vol. 65 (6), 1207-1214, https://doi.org/10.1093/jac/dkq075
- **IKAWA, S. ; TANI, A. ; NAKASHIMA Y. ; KITANO K.** Physicochemical properties of bactericidal plasma-treated water. *Journal of Physics D: Applied Physics,* 2016, vol. 49, 425401
- **JIANG, G. ; YUAN, Z.** Synergistic inactivation of anaerobic wastewater biofilm by free nitrous acid and hydrogen peroxide. *Journal of Hazardous Materials,* 2013, vol. 250-251, 91-98, https://doi.org/10.1016/j.jhazmat.2013.01.047
- **LAMMEL G. ; PERNER, D. ; WARNECK, P.** Decomposition of pernitric acid in aqueous solution. *Journal of Physical Chemistry,* 1990, vol. 94, 6141
- **LEE, Y.-N. ; LIND, J. A.** Kinetics of aqueous-phase oxidation of nitrogen(III) by hydrogen peroxide. *Journal of Geophysical Research,* 1986, vol. 91 (D2), 2793, https://doi.org/10.1029/JD091iD02p02793
- **LOEGAGER, T. ; SEHESTED, K.** Formation and decay of peroxynitric acid: a pulse radiolysis study. *The Journal of Physical Chemistry,* 2005, vol. 97 (39), 10047-10052
- **LOEGAGER, T. ; SEHESTED, K.** Formation and decay of peroxynitrous acid: a pulse radiolysis study. *The Journal of Physical Chemistry,* 1993, vol. 97 (25), 6664-6669
- **LÜBBERT C ; JOHN E ; VON MÜLLER L.** Clostridium difficile infection-guideline-based diagnosis and treatment. *Deutsches Ärzteblatt International,* 2014, vol. 111, 723
- **LUKES, P. ; DOLEZALOVA, E. ; CLUPEK, M. ; JABLONOWSKI, H. ; WOEDTKE, T. VON ; REUTER, S.** *Kinetics of peroxynitrite formation and its decomposition in air plasma treated liquids,* 2015, 5-8
- **SZABO, J. G. ; ADCOCK, N. J. ; RICE, E. W.** Disinfection of Bacillus spores with acidified nitrite. *Chemosphere,* 2014, vol. 113, 171-174, https://doi.org/10.1016/j.chemosphere.2014.05.038
- **VIONE, D. ; MAURINO, V. ; MINERO, C. ; BORGHESI, D. ; LUCCHIARI, M. ; PELIZZETTI, E.** New processes in the environmental chemistry of nitrite. 2. The role of hydrogen peroxide. *Environmental Science and Technology,* 2003, vol. 37 (20), 4635-4641, https://doi. org/10.1021 /es0300259